# EUROPEAN PATENT APPLICATION

(11) **EP 4 458 870 A1**
(43) Date of publication of application: **06.11.2024**
(21) Application number: 23171538.4
(22) Date of filing: 04.05.2023
(51) Int. Cl.: C08F 126/02, C08F 8/32, C08F 8/44

(54) **POLYMER DERIVATIVES AND THEIR USE AS LIPID NANOPARTICLE MODIFIERS**

(71) Applicant: Université de Liège, 4000 Liège (BE)
(72) Inventor: DEBUIGNE, Antoine, 4000 Liège (BE); PIEL, Géraldine, 4000 Liège (BE); MOTTET, Denis, 4000 Liège (BE); TOUSSAINT, Francois, 4000 Liège (BE); BERGER, Manon, 4000 Liège (BE); BEN DJEMAA, Sanaa, 4000 Liège (BE); LECHANTEUR, Anna, 4000 Liège (BE)
(74) Representative: V.O.

(57) **Abstract**

The present invention relates to a polymer derivative comprising a hydrophilic segment obtained from the polymerisation of N-methyl-N-vinylacetamide (PNMVA), coupled to at least one hydrophobic segment, said hydrophobic segment comprising at least one aliphatic chain of 8 to 28 carbon atoms. The present invention also relates to a method for preparing such polymer derivative. Lipid nanoparticles modified by PNMVA-based derivatives were prepared and advantages were shown compared to PEgylated lipid nanoparticles. The present invention also relates to the medical use of lipid nanoparticles containing PNMVA-based derivatives, particularly to the use of nucleic acid delivery, for example SiRNA delivery to target tumor cells.

## Description

### TECHNICAL FIELD OF THE INVENTION

The present invention relates to a polymer derivative and to a method for preparing such polymer derivative. The present invention also relates to a lipid nanoparticle comprising such polymer derivative and the use of such lipid nanoparticle in a medical treatment, in particular for nucleic acid delivery.

### BACKGROUND OF THE INVENTION

Nanomedicines are nanotechnology-based products for the administration of drugs. These are regarded as a promising strategy for the treatment of different diseases such as cancer or for vaccination. Nanomedicines use a nanocarrier for stable and efficient delivery of the active agent, such as small active pharmaceutical agents or nucleic acids. Lipid nanoparticles such as liposomes are the most common nanocarriers for delivery of active agents.

Liposomes are spherical, self-assembled structures formed by one or more concentric lipid bilayers composed of natural and/or synthetic lipids with an encapsulated aqueous phase in the center. Liposomes offer several advantages in delivering therapeutic molecules such as nucleic acids to cells. For example, liposomes can encapsulate or complex charged molecules (both negatively and positively charged), offer a degree of protection to the encapsulated or complexed molecule from degradative processes, and can be targeted to specific cells or tissues.

Liposomes are often modified with polyethylene glycol (PEG) to make them stealth, as described for example in Lechanteur et al., Eur. J. Pharm. Sci. 2016, 93, 493-503 and Berger et al., Int. J. Pharm. 2021, 605. Such PEGylation results in a reduction of the liposomes' affinity with phagocytes, so they can escape the recognition of the reticuloendothelial system and have a prolonged *in vivo* circulation time.

Although PEGylated lipid nanoparticles show many advantages, PEGylation has caused several problems: (i) the steric hindrance of PEG chain inhibits the uptake of liposomes by target cells; (ii) PEG interferes with the "endosomal escape" of liposomes, resulting in the degradation and failure of encapsulated drugs in the lysosome, these first two problems being commonly known as the PEG dilemma; (iii) repeated injection of PEGylated liposomes in the same subject induces the phenomenon of "accelerated blood clearance", known as ABC phenomenon, (iv) hypersensitivity reactions can occur and induce potentially severe allergic reactions.

There is therefore a significant need for a compound providing stealth properties to a lipid nanoparticle while avoiding one or more of the above problems, in particular avoiding the PEG dilemma and/or avoiding immunogenic reactions such as the ABC phenomenon and/or hypersensitivity.

### DESCRIPTION OF THE INVENTION

The inventors surprisingly found that polymer derivatives based on poly(N-methyl-N-vinylacetamide) (PNMVA) may solve one or more of above-mentioned problems.

The inventors surprisingly found that polymer derivatives based on N-methyl-N-vinylacetamide (NMVA) with at least one hydrophobic segment comprising at least one aliphatic chain of 8 to 28 carbon atoms may play a significant role for the modification of lipid nanoparticles, such as for example lipid nanoparticles dedicated to siRNA delivery.

It is an advantage that the polymer derivative of the invention has amphiphilic properties. It is an advantage that the polymer derivative of the invention is not cytotoxic. It is an advantage that the polymer derivative of the invention is not toxic to living organisms. It is an advantage that the polymer derivative of the invention is hemocompatible. It is an advantage that the polymer derivative of the invention can be inserted in a lipid nanoparticle.

It is an advantage that the lipid nanoparticles comprising the polymer derivative of the invention have good stealth properties. They may successfully avoid protein corona formation. It is an advantage that they have gene silencing efficiency. It is an advantage that they have an extended circulation time in a living body. It is a further advantage that they do not provoke inflammatory response in a living body. It is an advantage that they show a decreased ABC phenomenon compared to PEGylated nanoparticles. It is an advantage that they also show less immunogenic reaction. It is an advantage that they provide a good efficacy in siRNA delivery.

It is a first object of the invention to provide a polymer derivative comprising a hydrophilic segment obtained from the polymerisation of N-methyl-N-vinylacetamide, coupled to at least one hydrophobic segment, said hydrophobic segment comprising at least one aliphatic chain of 8 to 28 carbon atoms.

Polymer derivatives of the invention are constituted by a hydrophilic segment and a hydrophobic segment. This confers unique amphiphilic properties. For example, thanks to the hydrophobic segment, the polymer derivative can interact with lipid-based substances. Thanks to the hydrophilic segment, it can be soluble in aqueous environment.

The hydrophilic segment in the polymer derivate of the invention is a polymeric segment. By polymeric is meant obtained from the polymerization of a repeating unit (also referred to as a monomer). It is obtained from the polymerization of a vinyl monomer, which is N-methyl-N-vinylacetamide. It may also be referred to as poly(N-methyl-N-vinyl acetamide) or PNMVA. Advantageously, PNMVA may be hydrosoluble.

By polymeric segment is also meant a copolymer. A copolymer means it may be obtained from the polymerization of more than one repeating unit (different types of repeating units). For example, the hydrophilic segment may be obtained from the copolymerization of N-methyl-N-vinylacetamide and one or more other monomer(s). For example, from the copolymerization of N-methyl-N-vinylacetamide and N-methylvinylpyrrolidone.

The hydrophobic segment in the polymer derivate of the invention may for example be composed of a single aliphatic chain of 8 to 28 carbon atoms, which is saturated or unsaturated. Alternatively, the hydrophobic segment may be composed of 2-4 aliphatic chains which are all saturated, or all unsaturated or a combination of saturated and unsaturated. The hydrophobic segment may be composed of two aliphatic chains which are both saturated, or both unsaturated or one saturated and one unsaturated. The two or more aliphatic chains may have the same length or may have different lengths, as long as one of the aliphatic chain has a length of between 8 and 28 carbon atoms.

By aliphatic chain is meant a hydrocarbon chain, preferably said hydrocarbon chain is comprising only carbon and hydrogen atoms. Preferably, the aliphatic chain is an acyclic hydrocarbon chain. An aliphatic chain of between 8 and 28 carbon atoms enables an interaction with lipid-based substances. The interaction with lipid-based substances may be for example an insertion of the hydrophobic segment in a lipidic membrane.

Preferably, the aliphatic chain has a length of between 12 and 24 carbon atoms, more preferably between 14 and 20 carbon atoms, even more preferably between 16 and 18 carbon atoms.

Preferably, the polymer derivative comprises at least one hydrophobic segment which is chosen amongst the group of hydrophobic segments composed of a single aliphatic chain of 8 to 28 carbon atoms, two aliphatic chains of 8 to 28 carbon atoms, one or two fatty acids bearing an aliphatic chain of 8 to 28 carbon atoms, preferably of 12 to 24 carbon atoms, more preferably of 14 to 20 carbon atoms, even more preferably of 16 to 18 carbon atoms, a diglyceride bearing two aliphatic chains of 8 to 28 carbon atoms, preferably of 12 to 24 carbon atoms, more preferably of 14 to 20 carbon atoms, even more preferably of 16 to 18 carbon atoms, a ceramide bearing aliphatic chains of 8 to 28 carbon atoms, preferably of 8 to 24 carbon atoms, more preferably of 8 to 18 carbon atoms, even more preferably of 8 to 16 carbon atoms or a phospholipid bearing aliphatic chains of 8 to 28 carbon atoms, preferably of 12 to 24 carbon atoms, more preferably of 14 to 20 carbon atoms, even more preferably of 16 to 18 carbon atoms.

Preferably the hydrophobic segment is composed of at least one saturated aliphatic chain of 8 to 28 carbon atoms, preferably of 12 to 24 carbon atoms, more preferably of 14 to 20 carbon atoms, even more preferably of 16 to 18 carbon atoms.

Preferably the hydrophobic segment is a non-polymeric compound. By non-polymeric is meant that it is not obtained from the polymerization of one or more repeating units. Preferably the hydrophobic segment has up to 4 aliphatic chains wherein said aliphatic chains have 8 to 28 carbon atoms. This is advantageous for an efficient insertion in a lipidic membrane.

Preferably the at least one aliphatic chain of 8 to 28 carbon atoms is a terminal chain in the hydrophobic segment. By terminal chain is meant a position of the chain at an extremity of the hydrophobic segment which is not coupled with the hydrophilic segment. Again, this may favour insertion in a lipid-based substance.

A preferred phospholipid is a phosphatidylethanolamine bearing two aliphatic chains of 8 to 28 carbon atoms. Other types of phospholipids may also be used, such as for example phosphatidylcholine or phosphatidylserine, when comprising at least one aliphatic chain of 8 to 28 carbon atoms.

Examples include dodecyl, hexadecyl or octadecyl chain. It may for example coupled to the hydrophilic segment by a covalent bound. The hydrophobic segment may also be composed of a fatty acid. Preferred examples are stearic acid and palmitic acid. In other examples, the hydrophobic segment may comprise two aliphatic chains of from 8 to 28 carbon atoms. In such case, the length of the two aliphatic chains may be the same or different. Two aliphatic chains may for example be introduced in the form of a glycerol which is acylated in position 1 and 2 with fatty acids. Such compounds may also be referred to as diglycerides. Examples include 1,2-dimyristoyl-sn-glycerol (DMG), 1,2-dipalmitoyl-rac-glycerol (DPG), 1,2-distearoyl-rac-glycerol (DSG). Two aliphatic chains may also be introduced in the form of a phosphoethanolamine. Phosphoethanolamine bearing two aliphatic chains are commonly referred to as phosphatidylethanolamine. They belong to the family of phospholipids. Examples of phosphatidylethanolamine include 1,2-distearoyl-*sn*-glycero-3-phosphoethanolamine (DSPE), 1,2-dioleoyl-*sn-*glycero-3-phosphoethanolamine (DOPE), 1,2-dimyristoyl-*sn*-glycero-3-phosphoethanolamine (DMPE), 1,2-dipalmitoyl-*sn*-glycero-3-phosphoethanolamine (DPPE), 1,2-dilauroyl-*sn*-glycero-3-phosphoethanolamine (DLPE), 1,2-dioleoyl-*sn*-glycero-3-phosphoethanolamine (DOPE).

Preferably, the at least one hydrophobic segment is 1,2-distearoyl-*sn*-glycero-3-phosphorethanolamine (DSPE). DSPE has two saturated aliphatic chains of 18 carbon atoms.

PNMVA may advantageously have a controlled molecular weight. Such controlled molecular weight may be obtained from any suitable controlled polymerization process. For example, it can be obtained by a controlled radical polymerization process. For example, it can be obtained organometallic-mediated radical polymerization (OMRP), by tellurium-mediated radical polymerization (TERP) or reversible addition-fragmentation chain-transfer (RAFT) polymerization. A controlled polymerization process advantageous enables to couple a desired segment to the polymer, or to couple a function that will ease its coupling with a segment in a further step.

Coupling may for example be obtained through formation of a covalent bound. It can be an irreversible bound or a cleavable bound. A cleavable bound may be advantageous when it is desired to release the PNMVA chain from the hydrophobic segment. Cleavage may for example be obtained by an external stimulus, such as a light stimulus or by an internal stimulus, such as a pH change. Examples of possible bounds include an ether, ester, carbonate, amide or a urethane bound. Examples of possible cleavable bounds include imine, oxime or disulfur bound.

Advantageously, the functionalization or coupling rate is high, such as above 50% of coupling, preferably above 60 %, more preferably above 70%. A conversion rate may for example be determined by Nuclear Magnetic Resonance.

Advantageously, PNMVA has a molecular weight (Mₙ) below 50 000 g/mol, or below 20 000 g/mol, preferably between 1000 and 10 000 g/mol, more preferably between 1500 and 6000 g/mol, even more preferably between 2000 and 4000 g/mol. A molecular weight may for example be measured by size exclusion chromatography, optionally combined with multi-angle light scattering (MALLS).

Preferably, the hydrophilic segment has a molecular weight of between 1000 and 10 000 g/mol, preferably between 1500 and 6000 g/mol, even more preferably between 2000 and 4000 g/mol.

Advantageously, PNMVA has a low polydispersity (*Ð*). Polydispersity represents a molecular weight distribution range. It is obtained by the ratio between molecular weight in mass and molecular weight in number. Advantageously, the polydispersity is below 2, preferably below 1.5.

A second object of the invention is to provide a method for preparation of the polymer derivative recited above comprising the steps of:
(i) coupling a hydrophobic segment comprising at least one aliphatic chain of 8 to 28 carbon atoms as defined in the first object of the invention to a chain controlling agent;
(ii) polymerizing N-methyl-N-vinylacetamide in the presence of said coupled chain controlling agent and optionally of an initiator of radical polymerization or,
(i) polymerizing N-methyl-N-vinylacetamide in the presence of a chain controlling agent functionalized by a reactive group and optionally of an initiator of radical polymerization;
(ii) coupling the obtained polymer to a hydrophobic segment comprising at least one aliphatic chain of 8 to 28 carbon atoms as defined in the first object of the invention by reaction with said reactive group.

By chain controlling agent is meant a compound able to modulate the molar mass of the polymer chain and/or to functionalize the polymer chain. Functionalization is preferably at one extremity of the polymeric chain.

Preferably the chain controlling agent is a chain transfer agent.

By chain transfer agent is meant a compound able to functionalize NMVA growing chains during polymerization and/or to control the radical polymerization of NMVA. Suitable chain transfer agents include chain transfer agents known for RAFT polymerization, also called RAFT agents. In case of the use of a RAFT agent, an initiator of radical polymerization is added to polymerize N-methyl-N-vinylacetamide. Examples of RAFT agents are for example disclosed by Dupre-Demorsy et al. in Macromolecules, 2022, 55 (4), 1127. Advantageously, RAFT agents are able to control the radical polymerization of NMVA.

A RAFT agent may be represented by Formula I: 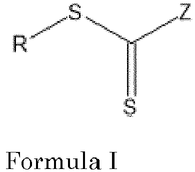
wherein Z is either O-R¹ (referred to as a dithiocarbonate or xanthate) or N-R₂R₃ (referred to as dithiocarbamate) with R¹, R², R³ each being separately one of an alkyl or a phenyl, substituted or not, preferably R¹ is methyl or an ethyl, preferably R₂R₃ are both unsubstituted phenyl group or both methyl;
and wherein R is any group capable of initiating polymerization.

Examples of RAFT agents are shown here after:

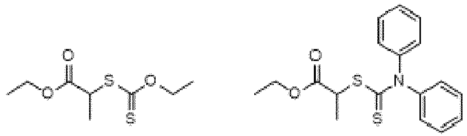

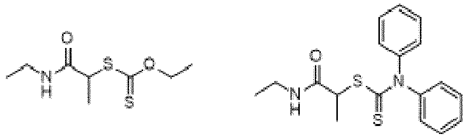

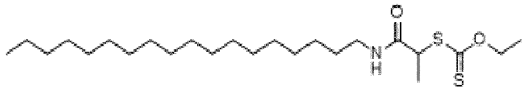

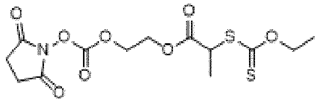

The chain transfer agent may advantageously be functionalized by a reactive group so as to introduce a function to the growing chains during polymerization, and hence to the obtained polymer. The function is preferably introduced at the end of the polymer chain. When the chain transfer agent is a xanthate species, such xanthate species may be functionalized. The reactive group may be for example an acid, an activated carbonate, an ester, a thiol, a carboxylic acid, an activated ester, N-hydroxysuccinimide ester (NHS esters), N-succinimidyl carbonate, an amine, an aldehyde, an alkyne, an azide.

By initiator of radical polymerization is meant any species able to generate a radical under the polymerization conditions. Preferred are azo initiators. Examples include 2,2'-azobis(4-methoxy-2.4-dimethyl valeronitrile) known as V70 or azobisisobutyronitrile (AIBN).

In order to obtain the polymer derivative, either the hydrophobic segment may be coupled to the chain transfer agent and the PNMVA chain is then growing from said segment, either a PNMVA chain is first obtained by polymerization, preferably controlled polymerization, and coupled to the hydrophobic segment in a second step. To enable such coupling, the chain transfer agent is preferably functionalized by a function enabling reaction with the desired hydrophobic segment. The reactive group may be as described above.

A third object of the invention is to provide a lipid nanoparticle comprising the polymer derivative recited above.

Thanks to its hydrophobic segment, the polymer derivative can be inserted within a lipid nanoparticle. The hydrophobic segment advantageously enables an interaction with the lipid part of the lipid nanoparticle, through the at least one aliphatic chain of from 8 to 28 carbon atoms.

On the other hand, the hydrophilic segment will at least partially extend out of the lipid nanoparticles, so as to interact with an aqueous environment of the lipid particle.

By lipid nanoparticle, one means spherical vesicles made of lipids. By lipid one means any molecule that has a hydrophobic character and is soluble in nonpolar solvents. Lipids may be of natural origin or may be of synthetic origin. Lipids include for example fatty acids, glycerides, phospholipids.

Lipid nanoparticles may particularly be made of neutral, ionic, ionizable or switchable lipids. They preferably bear a positive charge and/or are pH-sensitive, meaning that they may bear of positive charge at acidic pH while being neutral at basic/neutral pH. Examples of switchable lipids include CSL-3. Examples of pH sensitive lipid include SM-102. Examples of lipids also include cholesterol (CHOL), DOTAP, DOPE, DSPC, SPC, EPC, HSPC, HEPC, ceramides. Lipids used to form the nanoparticles may include one or more of the lipids used in the hydrophobic segment or may be different. A preferred composition is SPC/CHOL/polymer derivative or SM-102/DSPC/CHOL/polymer derivative or DOTAP/CHOL/DOPE/polymer derivative in different proportions.

Preferably the lipid nanoparticle has a mean diameter in the nanometer range.

A particularly interesting example of a suitable lipid nanoparticle is a liposome. A liposome is made of a phospholipid bilayer and an aqueous core. Cationic liposomes are liposomes containing at least one cationic lipid.

Preferably, the lipid nanoparticle may comprise the polymer derivative recited above in a molar percentage of from 1 to 50 %, preferably to 40 %, more preferably from 1 to 30% to the total of lipids.

By total lipid is meant the sum of the different lipids used in the preparation of the lipid nanoparticle. For example, three different lipids may be used in the preparation of the lipid nanoparticle. The molar percentage of each lipid is summed up to arrive at the total lipid. The molar percentage of the polymer derivative comprised in the lipid nanoparticle may depend from the use of the lipid nanoparticle. In particular, it may depend from the need of stealth behavior of the lipid nanoparticle. A longer circulation time in a living body, such as the targeting of tumor cells for example, will require a stealthier nanoparticle, hence a higher loading of polymer derivative. When used in a vaccine formulation, a lower loading may be sufficient (as low as 1.5 % for example).

Said polymer derivative may advantageously provide good steric protection of the lipid nanoparticle. Advantageously, it inhibits the adsorption of protein or particle on the surface of the lipid nanoparticle.

Preferably, the lipid nanoparticle comprising the polymer derivative as defined in the first object of the invention has a size of less than 500 nanometers, preferably less than 200 nanometers. The size is for example the hydrodynamic mean diameter of the lipid particle.

Advantageously, the lipid nanoparticle may further comprise an active agent.

By active agent, one means any agent having a cosmetic or therapeutic activity. An active agent may be a small pharmaceutical molecule, a biomolecule, a peptide-based agent, a protein or may be genetic material. Preferably, the active agent is a nucleic acid.

By nucleic acid, one means any of plasmids, mRNAs, miRNAs, ASO, morpholinos, siRNAs, shRNAs, small non coding RNAs, long non coding RNAs, rRNAs, CRISPR/Cas9 guides, catalytic oligos.

When the lipid nanoparticle is a cationic liposome and the active agent is a negatively charged nucleic acid, the resulting condensed particle may be referred to as a lipoplex.

When the lipid nanoparticle is composed of an ionizable or switchable lipid and the active agent is a negatively charged nucleic acid, the resulting condensed particle may be referred to as a Lipid Nanoparticle, LNP.

Advantageously the active agent may be encapsulated and/or complexed and/or solubilized by the lipid nanoparticle.

A fourth object of the invention is directed towards the lipid nanoparticle comprising the polymer derivative of the invention recited above for use in a method of medical treatment or of prevention of a disease.

The lipid nanoparticle comprising the polymer derivative of the invention is able to deliver an active agent to a subject in the need of a medical treatment. It may also deliver an active agent to a subject in the need of a prevention treatment. By prevention treatment is for example meant a vaccine.

The lipid nanoparticle comprising the polymer derivative of the invention may be particularly for use in drug delivery.

The lipid nanoparticle comprising the polymer derivative of the invention may be loaded with a suitable substance for example to target tumor cells. Before it reaches its target, the active agent will be protected by the lipid nanoparticle. Thanks to its stealth properties conferred by the hydrophilic PNMVA segment, the formation of a protein corona is avoided. Moreover, an efficient delivery in the target is made possible. Indeed, the lipid nanoparticle comprising the polymer derivative of the invention enables a higher cellular uptake compared to the same lipid nanoparticles that were PEGylated. For example, lipid nanoparticles comprising the polymer derivative of the invention were up to 6 times more internalized than their PEG equivalent when tested on human lung adenocarcinoma cells A549.

Repeated injections in mouse also showed that the lipid nanoparticles comprising the polymer derivative of the invention are less immunogenic than PEGylated equivalents. A significantly lower production of IgG antibody was shown after second injection and no increase in IgM antibody was observed. No ABC phenomenon was observed as is the case with PEGylated equivalents.

Furthermore, the lipid nanoparticles comprising the polymer derivative of the invention are safe to use. No accumulation in liver nor spleen was observed. No inflammation (production of pro-inflammatory cytokines) was observed. The safety use of the derivatives and of the lipoplexes incorporating these derivatives was also shown, at the cellular level, in animal models of zebrafish and mice and on human blood.

Due to all these properties, the lipid nanoparticles comprising the polymer derivative of the invention may advantageously be used in any application where PEGylated lipid nanoparticles are currently used. An example is Doxil^{®}, the first liposome drug approved by the US FDA. It contains doxorubicin as active agent, an agent for chemotherapy. A second example of currently used PEGylated lipid nanoparticle is the excipient in both the Comirnaty^{®} and Spikevax^{®} COVID-19 vaccine. The active agent in both vaccines is mRNA. A third example is Onpattro, a lipid nanoparticle-based short interfering RNA drug for the treatment of polyneuropathies, with liver targeting.

In particular, when the lipid nanoparticle comprising the polymer derivative of the invention as recited above comprises a nucleic acid which may be used in nucleic acid delivery. For example, it may be used to deliver nucleic acid to targeted cells, such as tumor cells. It may be used for a medical treatment but also for the prevention of a medical disease, as in a vaccine administration.

The amount of nucleic acid versus polymer derivatives may be expressed as the N/P ratio, N and P corresponding respectively to the number of moles of positive charges in the lipid nanoparticle and negative charges in the nucleic acid. The number of moles of positive charges is for example based on the cationic lipid present, for example an ammonium-based lipid, for example DOTAP. This enables the complexation of negatively charged nucleic acid with positively charged lipid nanoparticle. The N/P ratio may vary according to the genetic material. When siRNA is used, an N/P ratio of 1 to 10 is preferably used, more preferably from 2 to 5, for example 2.5.

Lipid nanoparticles with polymer derivatives of the invention may advantageously enhance the cell uptake of lipoplexes compared to PEGylated lipoplexes. Preferably cell uptake is favored when the PNMVA chain is relatively short, for example, when the molecular weight of PNMVA is from 2000 to 4000 g/mol. Advantageously, lipoplexes containing siRNA and grafted with polymer derivatives having PNMVA of 2500 g/mol may be about 6 times more internalized than the PEGylated lipoplexes, when tested on A549 cells.

The lipid nanoparticle comprising the polymer derivative of the invention may be administered by any suitable administration method, such as an oral administration, a cutaneous administration, an injection, an intravenous administration, a subcutaneous administration, a systemic administration, a parenteral, digestive, vaginal, rectal or transcutaneous administration, a transmucosal administration, a pulmonary, nasal or sublingual administration, an inhalation or a nebulization or an aerosol administration. Preferably, the composition is administered by parenteral injection administration.

Thanks to the hydrophilisation of the lipid nanoparticles by the polymer derivative of the invention, an improvement of their mucopenetration is expected. This property can be used for example for pulmonary or vaginal administration.

A further object of the invention is directed towards the use of the lipid nanoparticle comprising the polymer derivative of the invention as recited above for a cosmetic treatment.

The lipid nanoparticle comprising the polymer derivative of the invention may be loaded with any active agent providing a cosmetic treatment, such as a cosmetic treatment of the skin for example. In that case, it will be preferably administrated in a cream or any suitable topical administration or an oral administration.

Another object of the invention is to provide a pharmaceutically composition comprising the lipid nanoparticle comprising the polymer derivative of the invention as recited above and a pharmaceutically acceptable carrier.

A yet further object of the invention is to provide a method for preparing the lipid nanoparticle comprising the polymer derivative of the invention as recited above comprising the steps of:
(i) providing a lipid nanoparticle;
(ii) optionally adding an active agent, preferably an active agent selected from a nucleic acid so as to form charge interaction between the lipid nanoparticle and the active agent;
(iii) adding a polymer derivative of the invention under stirring in a solvent;
(iv) optionally removing the solvent.

Preferably, the solvent is an aqueous solvent. Preferably, the solvent is water.

Preferably removing the solvent is performed by filtering, evaporation, centrifugation, lyophilization, and/or spray drying.

In an alternative method, all ingredients may be added at once.

Alternatively, the method may comprise the steps of:
(i) solubilising lipids and optionally an active agent when such active agent is a hydrophobic compound in a first solvent so as to form a first solution,
(ii) optionally solubilising an active agent when such active agent is a hydrophilic compound in a second solvent so as to form a second solution,
(iii) optionally mixing the first solution and the second solution,
(iv) removing the first solvent and optionally the second solvent,
wherein a polymer derivative of the invention is added in step (i) or in step (ii) or after step (iv).

The steps are presented in an order which is solely indicative. The order of the steps may be varied without departing from the scope of the invention. Some of the steps may also be repeated without departing from the scope of the invention.

When the polymer derivative is added after step (iv), it is preferably added under stirring in an aqueous solution.

Preferably at least one active agent is a nucleic acid. Preferably nucleic acid is considered as a hydrophilic compound and is solubilised in step (ii).

Preferably step (iii) is a continuous mixing.

Preferably step (iii) is performed in a microfluidic equipment (also referred to as rapid mixing).

By microfluidic equipment is meant equipment having channels with a diameter in the micrometer range, equal to or less than 1 mm.

Advantageously, the first solvent is a solvent capable of solubilising all substances provided in step (i) so as to form a first solution. Preferably, the first solvent is an organic solvent miscible with water. More preferably the first solvent is ethanol. Alternatively the first solvent is chloroform.

Advantageously, the second solvent is a solvent capable of solubilising a nucleic acid so as to form a second solution. Preferably the second solvent is an aqueous solution. More preferably the second solvent is water.

Advantageously the first solution and the second solution are mixed continuously in a microfluidic equipment. Preferably, the flow rate of each solution is adapted according to the desired N/P ratio.

Advantageously, the first solvent and/or the second solvent is a supercritical fluid. Preferably the supercritical fluid is carbon dioxide (CO₂). When the supercritical fluid is gaseous at ambient temperature, as is the case for CO₂, it may advantageously be removed by depressurisation.

### BRIEF DESCRIPTION OF THE DRAWINGS

Figure 1: Z-average diameter in nanometer (nm) of naked lipoplexes versus grafted lipoplexes (grafting by DSPE-PEG, by DSPE-PNMVA24 and by DPSE-PNMVA50 in N/P ratio of 2.5, 100 nM, at increasing percentages (10, 15, 30, 40%) in molar ratio of polymer derivative versus total lipids.
Figure 2: Polydispersity index (PdI) of naked lipoplexes versus grafted lipoplexes.
Figure 3: Zeta Potential (Zp) (mV) of naked lipoplexes versus grafted lipoplexes.
Figure 4: siRNA complexation (%) of naked lipoplexes versus grafted lipoplexes.
Figure 5: Δ*f* traces (7^{th} harmonic) from QCM-D monitoring of the deposition of (a) Et-PNMVA₂₇ (b) OD-PNMVA₃₁, (c) DSPE-PNMVA₂₄ and (d) DSPE-PNMVA₅₀.
Figure 6: Mean size increase of naked lipoplexes and grafted lipoplexes mean size after 2 h in fetal bovine serum (30%).
Figure 7: Cell viability of A549 cell line treated for 24 h with polymer derivatives of the invention and with comparative DPSE-PEG.
Figure 8: Cell viability of A549 cell line treated for 24h with naked lipoplexes and grafted lipoplexes.
Figure 9: Fluorescence intensity of A549/GFP cells in presence of naked and grafted lipoplexes
Figure 10: Gene knockdown (MFI, %) in A549/GFP cells when transfected with lipoplexes carrying anti-GFP siRNA (100 nM) compared with naked lipoplexes, lipoplexes grafted with DSPE-PEG and with Lipofectamine^{®} RNAiMAX (black line) after 72 h of transfection (n=3).
Figure 11: Fluorescence intensity of grafted lipoplexes containing fluorescent Cy5.5 GL3 SiRNA after a first (5h) and a second (24h) injection into mice, using *in vivo* imaging.
Figure 12: Fluorescence intensity of grafted lipoplexes containing containing fluorescent Cy5.5 GL3 SiRNA using *ex vivo* imaging in kidney, heart, liver, lung and spleen.
Figure 13: Level of IgM against grafted lipoplexes one week after a first and a second injection, using ELISA assay.
Figure 14: Level of IgG against grafted lipoplexes one week after a first and a second injection, using ELISA assay.

### DESCRIPTION OF ILLUSTRATIVE EMBODIMENTS

The invention will now be described with respect to particular embodiments and with reference to certain drawings. It is clear that other embodiments of the invention can be configured according to the knowledge of skilled persons in the art without departing from the technical teachings of the invention, the invention being only limited by the claims.

### 1. Synthesis of PNMVA Derivatives

To impart amphiphilicity to PNMVA and allow its possible anchoring into the membrane of the lipoplexes, this hydrosoluble polymer sequence was end-functionalized with two different hydrophobic groups, namely octadecyl (OD) and 1,2-distearoyl-sn-glycero-3-phosphoethanolamine (DSPE). These will be further referred to as OD-PNMVA and DSPE-PNMVA respectively (Scheme 1).

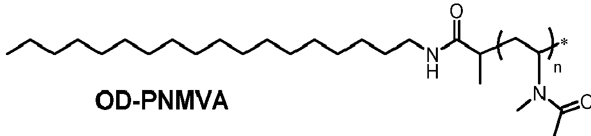

The synthesis strategy of these polymer derivatives is shown in Scheme 2 and Scheme 3 respectively. Both schemes involve reversible addition fragmentation chain transfer (RAFT) polymerization.

The synthesis of the OD-PNMVA was achieved by RAFT polymerization of NMVA by using an OD-containing RAFT agent. The synthesis of the DSPE-PNMVA derivatives was achieved by a two step-process based on (i) RAFT of NMVA initiated by a xanthate containing a succinimidyl carbonate (SC-XA) and (ii) coupling of the resulting succinimidyl carbonate-PNMVA (SC-PNMVA-XA) with the amino group of DSPE. The DP of PNMVA and so the molar mass of the hydrophilic part, was adjusted by the monomer/RAFT agent ratio.

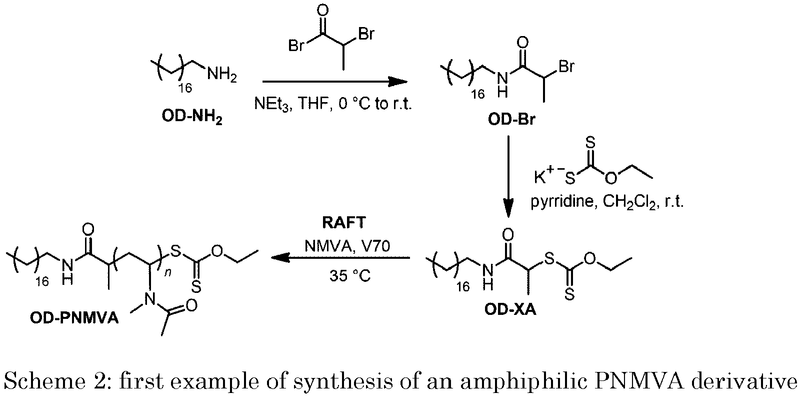

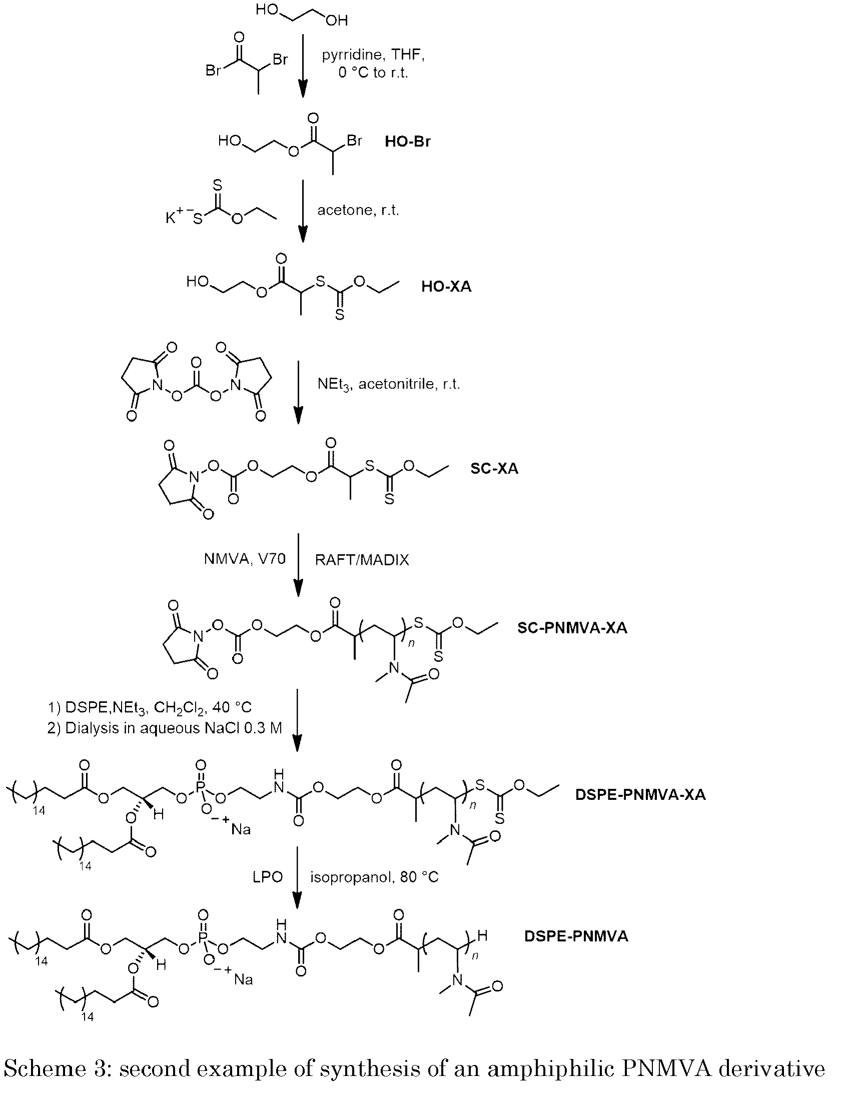

The materials used was as follows: potassium ethyl xanthogenate (96%, Aldrich), octadecylamine (97%, Aldrich), 2-bromopropionyl bromide (97%, Aldrich), N,N'-disuccinimidyl carbonate (DSC) (Aldrich), 1,2 distearoylphosphatidylethanolamine (DSPE)(Aldrich), ethylene glycol (Roth), magnesium sulfate (Abcr), ammonium chloride (Aldrich), sodium bicarbonate (Aldrich), lauroyl peroxide (Fluka), 2,2'-azobis(4-methoxy-2,4-dimethylvaleronitrile) (V70, t½ = 10 h at 30 °C) (Wako), hydrochloric acid (Acros), potassium phosphate monobasic (Janssen Chimica), potassium phosphate dibasic (VWR), sodium chloride (VWR), 1,2-dioleoyl-3-trimethylammonium-propane chloride salt (DOTAP) (Avanti polar lipids), 1,2-dioleoyl-sn-glycero-3-phosphoethanolamine (DOPE) (Avanti polar lipids), cholesterol (Aldrich), ammonium peroxide (Aldrich), 3-mercaptopropionic acid (MPA) (Aldrich), hydrogen peroxide (30%, Aldrich), sulfuric acid (95-97%, Merck), silica gel for column chromatography (60 Å, ROCC S.A.), triethylamine (99%, Acros organic), pyridine (Aldrich), diethyl ether (VWR), n-hexane (> 99%, VWR), ethyl acetate ( > 99.9%, VWR), acetonitrile ( > 99.99 %, Fisher), heptane (>97.5%, Fisher), absolute ethanol (Fisher) and isopropanol (Fisher) were used as received. N-methyl-N-vinylacetamide (Aldrich) was dried over calcium hydride, degassed and distilled under reduced pressure prior to use. *N*,*N*-dimethylformamide (DMF, > 99%, VWR) was dried over molecular sieves and degassed prior to use. Dichloromethane (CH₂Cl₂) was degassed and dried over 4 Å molecular sieves. For the characterization of the samples by quartz crystal microbalance, QCM-D sensors were gold coated, AT-cut quartz crystals with a fundamental frequency of about 5 MHz (Q-Sense, Sweden).

In order to characterize the sample, following methods were used. Polymer derivatives were analyzed by size exclusion chromatography (SEC) in DMF containing 0.025 M of LiBr at 55 °C with a Waters chromatograph equipped with three columns (Waters Styragel PSS GRAM 1000Ǻ (×2), 30Ǻ), a dual λ absorbance detector (Waters 2487) and a refractive index detector (Waters 2414). The system was operated at a flow rate of 1 mL/min. The molar mass of the SC-PNMVA-XA was determined by SEC equipped with a multiangle laser light scattering (MALLs) detector in DMF/LiBr (0.025 M). The Wyatt MALLs detector (120 mW solid-state laser, k 1/4 658 nm, DawnHeleos S/N342-H) measures the excess Rayleigh ratio Rh (related to the scattered intensity) at different angles for each slice of the chromatogram. The specific refractive index increment (dn/dc) of polymer was measured by using a Wyatt Optilab refractive index detector (k 1/4 658 nm). Data were processed with the Astra V software (Wyatt Technology). ¹H and ¹³C nuclear magnetic resonance (NMR) were recorded at 298 K with a Bruker AVANCE III HD spectrometer (B0 = 9.04 T) (400 MHz) and treated with MestreNova software.

**2-bromo-N-octadecylpropanamide (OD-Br)** was obtained as follows. Octadecylamine (3.00 g, 11.1 mmol) and triethylamine (1.68 g, 16.6 mmol) were dissolved in 100 mL of dried and degassed THF under nitrogen into a 250 mL two-necked round-bottom flask equipped with a three-way stopcock and an addition funnel containing 2-bromopropionyl bromide (2.87 g, 13.3 mmol) in 10 mL of dried and degassed THF. The octadecyl amine solution was cooled to 0 °C before dropwise addition of the 2-bromopropionyl bromide solution. The reaction medium was stirred 30 minutes at 0 °C and overnight at room temperature. A white precipitate was filtered off and the solvent was evaporated under vacuum. The crude product was solubilized in ethyl acetate/hexane 3/1 v/v (40 mL) at 50 °C and the mixture was stored overnight at 6 °C. White crystals of 2-bromo-N-octadecylpropanamide were formed, filtered and dried under vacuum (3.7 g, 83 % yield). ¹H NMR (400 MHz, CDCl₃): δ[ppm] = 6.37 (1H, s, C(O)NH), 4.41 (1H, q, J=7.1 Hz, CH), 3.26 (2H, q, C(O)NHCH₂), 1.88 (3H, d, J=7.1 Hz, CHCH₃), 1.52 (2H, m, C(O)NHCH₂CH₂), 1.27 (30H, m, (CH₂)₁₅), 0.88 (3H, t, J=7.0 Hz, CH₃(CH₂)₁₅). ¹³C NMR (100.6 MHz, CDCl₃): δ[ppm] = 169.12 (C=O), 45.67 (CH), 40.23 (C(O)NHCH₂), 31.93, 29.71, 29.68, 29.67, 29.64, 29.57, 29.51, 29.37, 29.28, 29.25, 26.81, 23.34 (CHCH₃), 22.70, 14.13 (C(S)OCH₂CH₃).

**O-ethyl S-(1-(octadecylamino)-1-oxopropan-2-yl) carbonodithioate (OD-XA)** was obtained as follows. OD-Br (5.00 g, 12.3 mmol) and potassium ethyl xanthogenate (7.90 g, 49.3 mmol) were placed in a 1 L flask under nitrogen and added with a degassed solution of pyridine (65.2 g, 824 mmol) in dried and degassed dichloromethane (330 mL). The reaction mixture was stirred at room temperature for 24 h. Dichloromethane (100 mL) was added to the solution before consecutive washes with saturated NH₄Cl (three times with 50 mL), NaHCOs (three times with 50 mL) and water (three times with 50 mL). The organic layer was dried over magnesium sulfate, filtered and evaporated under vacuum. The crude product was solubilized in ethyl acetate/hexane 3/2 v/v (40 mL) and stored at 6 °C overnight. Pale yellow crystals of O-ethyl S-(1-(octadecylamino)-1-oxopropan-2-yl) carbonodithioate were formed, filtered and dried under vacuum (4.37 g, 79 % yield). ¹H NMR (400 MHz, CDCl₃): δ[ppm] = 6.33 (1H, s, C(O)NH), 4.64 (2H, q, J=7.2 Hz, C(S)OCH₂), 4.27 (1H, q, J=7.4 Hz, CH), 3.22 (2H, q, J=7.0 Hz, C(O)NHCH₂), 1.54 (3H, d, J=7.4 Hz, CHCH₃), 1.46 (2H, m, C(O)NHCH₂CH₂), 1.41 (3H, t, J=7.2 Hz, C(S)OCH₂CH₃), 1.24 (30H, m, (CH₂)₁₅), 0.87 (3H, t, J=7.0 Hz, CHs(CH₂)₁₅). ¹³C NMR (100.6 MHz, CDCl₃): δ[ppm] = 213.83 (C=S), 170.83 (C=O), 70.85 (C(S)OCH₂), 48.17 (CH), 39.97 (C(O)NHCH₂), 32.07, 29.85, 29.82, 29.80, 29.72, 29.69, 29.53, 29.51, 29.40, 26.99, 22.84, 16.63 (CHCH₃), 14.26 (CH₃(CH₂)₁₅), 13.88 (C(S)OCH₂CH₃).

**Synthesis of OD-PNMVA.** OD-XA (703 mg, 1.58 mmol) as chain transfer agent and V70 (487 mg, 1.58 mmol) as initiator were placed under inert atmosphere in a Schenck tube and added with distilled, dried and degassed NMVA (7.8 g, 79 mmol). The reaction mixture was then stirred at 35 °C. After 6h, the reaction was stopped and the monomer conversion measured by ¹H NMR in CD₂Cl₂ reached 50 %. The mixture was diluted in dichloromethane followed by precipitation in diethylether. The polymer was recovered by filtration, dried under vacuum at 40 °C for 12 h. The polymer was further purified by dialysis in acetone through a 1 kDa porous membrane for 24 h and in miliQ water through a 500 Da porous membrane for 12h, followed by lyophilization. The desired OD-PNMVA₃₁ (0.98 g) was recovered as a pale yellow powder and characterized by SEC in DMF using a PS calibration (*M*_{n SEC} = 1800 g/mol, *Ð* =1.21) and ¹H NMR in CD₂Cl₂ (*M*_{n NMR PNMVA} = 3100 g/mol, DP = 31).

**Synthesis of 2-hydroxyethyl 2-bromopropionate (HO-Br).** Dried ethylene glycol (4.84 mol, 300 g, 271 mL) and pyridine (0.1 mol, 7.91 g, 8.1 mL) were diluted with 100 mL dried THF in a 1 L two-necked round-bottom flask equipped with an addition funnel under inert atmosphere. 2-Bromopropionyl bromide (95 mmol, 20.51 g, 9.95 mL) was placed in the addition funnel with 50 mL dried THF. The flask was cooled in an ice bath and the 2-bromopropionyl bromide was added dropwise and the mixture was stirred at 0 °C for 1h. The mixture was then stirred at room temperature for 16 h. After this, the mixture was poured into 800 mL acidic water (pH = 2, hydrochloric acid) and the product was extracted with dichloromethane (6 × 100 mL). The organic fractions were combined, extracted with water and dried over magnesium sulfate. Solvent was evaporated and product was obtained as a colourless liquid (MM = 197.03 g/mol, 14.92 g, yield = 80%). ¹H NMR (400 MHz, CDCl₃): δ[ppm] = 4.40 (1H, s, J=7.0 Hz, CH), 4.28 (2H, m, CH₂OC(O)), 3.84, (2H, m, HOCH₂CH₂OC(O)), 2.27 (1H, s, OH), 1.82 (3H, d, J= 7 Hz, CH₃CH).¹³C NMR (100.6 MHz, CDCl₃): δ[ppm] = 170.59 (C=O), 67.45 (CH₂OC(O)), 61.00 (HOCH₂CH₂OC(O)), 39.89 (CH), 21.66 (CH₃CH).

**S-(1-Methyl-4-hydroxyethyl acetate)O-ethyl dithiocarbonate (HO-XA).** Potassium ethyl xanthogenate (3.65 g, 22.7 mmol) was placed with 15 mL acetone under inert atmosphere in a round-bottom flask equipped with an addition funnel containing 2-hydroxyethyl 2-bromopropionate (4.00 g, 20.3 mmol) in 15 mL acetone. The latter solution was added dropwise at room temperature over 30 min and the reaction mixture was stirred at room temperature for 24 h. After filtration of a white solid and washing with 50 mL of acetone, the solvent was evaporated. The residue was then dissolved in 50 mL dichloromethane and extracted with distilled water (3 × 25 mL). The organic phase was dried over magnesium sulfate, filtered and evaporated to dryness. The desired product was obtained as a viscous yellow liquid (2.95 g, 61% yield). ¹H NMR (400 MHz, CDCl₃): δ[ppm] = 4.59 (2H, q, J= 7.1 Hz, C(S)OCH₂), 4.37 (1H, q, J= 7.4 Hz, CH), 4.23 (2H, m, CH₂OC(O)), 3.79 (2H, t, J= 4.7 Hz, HOCH₂CH₂OC(O)), 2.32 (1H, s, OH), 1.55 (3H, d, J= 7.3 Hz, CH₃CH), 1.37 (3H, t, J= 7.2 Hz, C(S)OCH₂CH₃). ¹³C NMR (100.6 MHz, CDCl₃): δ[ppm] = 212.38 (C=S), 171.85 (C=O), 70.55 (C(S)OCH₂), 67.32 (CH₂OC(O)), 61.06 (HOCH₂CH₂OC(O)), 47.24 (CH), 16.80 (CH₃CH), 13.77 (C(S)OCH₂CH₃).

**2-((((2,5-dioxopyrrolidin-1-yl)oxy)carbonyl)oxy)ethyl 2-((ethoxycarbonothioyl)thio)propanoate (SC-XA).** HO-XA (1.00 g, 4.19 mmol) and N,N-disuccinimidyl carbonate (2.13 g, 8.32 mmol) were placed under nitrogen atmosphere in a 25 mL round bottom flask with triethylamine (2.13 g, 21.03 mmol) and 10 mL acetonitrile. The mixture was stirred at room temperature for 24 h. The reaction medium was then diluted with 20 mL dichloromethane followed by solvent evaporation to facilitate the removal of acetonitrile. The resulting viscous brown liquid was then diluted with 10 mL of dichloromethane and washed with aqueous NaHCO₃ (3x25 mL) and distilled water (3x25mL). The organic phase was dried over magnesium sulfate, filtered and evaporated to dryness. SC-XA was obtained as a brown viscous liquid (1.04 g, 65% yield). ¹H NMR (400 MHz, CDCl₃): δ[ppm] = 4.63 (2H, q, J=7.1 Hz, C(S)OCH₂), 4.53 (2H, t, J=4.8 Hz, CH₂OC(O)), 4.48-4.34 (3H, m, CH+ C(O)O*CH*₂CH₂OC(O)CH), 2.84 (4H, m, NC(O)(CH₂)₂C(O)N), 1.58 (3H, d, J=7.4 Hz, CH₃CH), 1.41 (3H, t, J=7.2 Hz, C(S)OCH₂CH₃). ¹³C NMR (100.6 MHz, CDCl₃): δ[ppm] =211.75 (C=S), 171.29 (C(O)CH), 168.48 (C(O)NC(O)), 151.51 ((O)C(O)), 70.40 (C(S)OCH₂), 68.35 (CH₂OC(O)), 62.41 (C(O)O*CH*₂CH₂OC(O)CH), 46.85 (CH), 25.47 (NC(O)(CH₂)₂C(O)N), 16.56 (CH₃CH), 13.67 (C(S)OCH₂CH₃).

**Synthesis of SC-PNMVA-XA.** SC-XA (519 mg, 1.36 mmol) and V70 (420 mg, 1.36 mmol) were placed a under inert atmosphere in a Schenck tube and added with distilled, dried and degassed NMVA (6.8 g, 68.1 mmol). The reaction mixture was then stirred at 35 °C. After 5h, the reaction was stopped and the monomer conversion measured by ¹H NMR in CDCl₃ reached 70 %. The mixture was diluted in dichloromethane and the polymer was purified twice by precipitation in diethyl ether. After drying under vacuum at 40 °C for 24 h, SC-PNMVA₄₀-XA (2.71 g) was collected as a pale yellow powder and characterized by SEC in DMF using a PS calibration (*M*_{n, SEC} = 3800 g/mol, *Ð* = 1.20) and MALLS (dn/dc = 0.071) (*M*_{n, MALLS} = 4300 g/mol, *Ð* = 1.16). This procedure was adapted for the production of SC-PNMVA₃₀-XA.

**Table 1. Synthesis of succinimidyl carbonate-PNMVA derivatives**

| Compounds | Tim e (h) | Conv.^{a} (%) | *M*_{n,th}^{b} (g/mol) | *M*_{n, SEC^{c}} (g/mol) | *Ð^{c}* | *M*ₙ, _{MALLS}^{d} (g/mol) | *Ð^{d}* |
|---|---|---|---|---|---|---|---|
| SC-PNMVA₃₀-XA | 4 | 65 | 3600 | 3000 | 1.12 | 3400 | 1.17 |
| SC-PNMVA₄₀-XA | 6.5 | 70 | 3800 | 3800 | 1.20 | 4300 | 1.16 |

Conditions: [NMVA]₀/[SC-XA]₀/[V70]₀ = 50/1/1, 35 °C, in bulk. ^{a} Determined by ¹H NMR in CDCl₃ on the crude mixture by integrating the signals of the methyl group of the monomer (at 3.08 ppm, 3H) and the signals of the methyl group of the polymer (at 2.73 ppm, 3H). ^{b} *M*_{n, th} = [NMVA]₀/[CTA]_{0 ×} conv. _{×} MM _{NMVA} + MM_{SC-XA}. ^{c} *M*_{n, SEC} determined by SEC DMF/LiBr (PS calibration) on the crude mixture. ^{d} Determined by MALLS in DMF/LiBr (dn/dc = 0.071 mL.g⁻¹) after purification of the polymer by precipitation two times in diethyl ether and drying under vacuum at 40 °C.

**Synthesis of DSPE-PNMVA-XA.** SC-PNMVA₄₀-XA (2 g, 5.88 mmol), DSPE (440 mg, 5.88mmol) and triethylamine (2.5 g, 24.57 mmol) were placed in a 25 mL round-bottom flask under nitrogen atmosphere and dissolved in dried and degassed dichloromethane (10 mL). The mixture was then stirred at 40 °C for 1 h. The solvent was then evaporated and the residue dissolved in acetonitrile (12 mL) and stored at 6 °C overnight. The solution was then centrifugated two times (10 000 rpm, 15 min, 6 °C). The supernatant was collected and evaporated under vacuum affording a white powder. The latter was then diluted in milliQ water and dialyzed through a 1kDa porous membrane sequentially against an aqueous sodium chloride solution (0.3M) for 12 h and distilled water for 6 h. After lyophilization, the desired DSPE-PNMVA₅₃-XA (1.8 g) was obtained as a white powder and characterized by SEC in DMF using a PS calibration (*M*_{n, SEC} = 5000 g/mol, *Ð* = 1.12) and ¹H NMR in CDCl₃ (*M*_{n, NMR} = 5300 g/mol, DP = 53). This procedure was adapted for the production of DSPE-PNMVA₃₅-XA.

**Table 2. Synthesis of DSPE-PNMVA-XA via coupling of DSPE and SC-PNMVA-XA.**

| Final compound | SC-PNMVA precursor | *M*_{n, NMR} ^{a} (g/mol) | *Ð^{b}* | DP ^{c} |
|---|---|---|---|---|
| DSPE-PNMVA₃₅-XA | SC-PNMVA₃₀-XA | 3400 | 1.12 | 35 |
| DSPE-PNMVA₅₃-XA | SC-PNMVA₄₀-XA | 5300 | 1.12 | 53 |

Conditions: [SC-PNMVA-XA]₀/[DSPE]₀/[NEt₃]₀ = 1/1/4.2, 40 °C, 1h. ^{a} *M*_{n, NMR DSPE-PNMVA-XA} determined by ¹H NMR in CDCl₃ on the purified polymer based on the relative intensities of the signal of the α-terminal methyl groups (at 0.87 ppm, 6H) and the methyl signal of PNMVA (2.5-2.9 ppm, 3H). ^{b} Determined by SEC DMF/LiBr (PS calibration) after purification of the polymer by precipitation in acetonitrile, centrifugation and solvent evaporation. ^{c} Degree of polymerization of the PNMVA segment deduced from NMR.

**Removal of the terminal xanthate.** The above mentioned DSPE-PNMVA₅₃-XA (1.2 g, 0.2 mmol) and LPO (33.5 mg, 0.084 mmol) were placed under inert atmosphere in a 10 mL round-bottom flask and dissolved in degassed isopropanol (5 mL). The mixture was then stirred at 80 °C for 8h during which LPO portions (16.8 mg, 0.042 mmol) were added every 2 h. The mixture was stirred for at 80 °C for an additional 16h. The polymer was recovered by precipitation in hexane, dried under vacuum at 40 °C for 12 h and dialyzed through a 1kDa porous membrane sequentially against an aqueous sodium chloride solution (0.3 M) for 12h and against water for 12h. After lyophilization, the desired DSPE-PNMVA₅₀-H was collected as a white powder (480 mg). The absence of the xanthate was confirmed by SEC-UV by the disappearance of the xanthate's characteristic absorption signal at 290 nm. This procedure was also applied to DSPE-PNMVA₃₅-XA leading to DSPE-PNMVA₂₄-H.

**Table 3. DSPE-PNMVA derivatives obtained after terminal xanthate removal.**

| Final compound | DSPE-PNMVA-XA precursor | *M*_{n, SEC} ^{a} (g/mol) | *Ð ^{a}* | *M*_{n, NMR} ^{b} (g/mol) | DP ^{c} |
|---|---|---|---|---|---|
| DSPE-PNMVA₅₀-H | DSPE-PNMVA₅₃-XA | 2800 | 1.27 | 5000 | 50 |
| DSPE-PNMVA₂₄-H | DSPE-PNMVA₃₅-XA | 1800 | 1.28 | 2400 | 24 |

Conditions: [DSPE-PNMVA-XA]₀/[LPO]₀= 1/1.2, 80 °C in isopropanol, 24 h. ^{a} Determined by SEC DMF/LiBr (PS calibration) after purification of the polymer by precipitation in hexane and successive dialysis in water and 0.1M NaCl. ^{c} *M*_{n, NMR, PNMVA} determined by ¹H NMR in CDCl₃ on the purified polymer based on the relative intensities of the signal of the α-terminal methyl groups (at 0.87 ppm, 6H) and the methine signal of PNMVA (4.0-4.7 ppm, 1H). ^{c} Degree of polymerization of the PNMVA segment deduced from NMR.

**Table 4** summarizes the PNMVA structures prepared accordingly and tested to decorate lipid nanoparticles (also referred to as lipoplexes) in the next sections. Et-PNMVA₂₇ is a PNMVA modified with an ethyl chain, hence only 2 carbon atoms. It was used as a comparative example. All polymers were purified by repeated precipitation, prolonged dialysis in water and lyophilization. In the case of the DSPE-PNMVA derivatives, the terminal XA removal was achieved by treatment with lauroyl peroxide (LPO) at 80 °C in isopropanol. Proper elimination of the XA terminal group of DSPE-PNMVAwas confirmed by the disappearance of the xanthate characteristic absorption peak at 290 mm in the size exclusion chromatography-UV curves.

**Table 4. Characteristics of the PNMVA derivatives.**

| **Polymer** | **DP^{a}** | ***M*_{n NMR}^{b} (g/mol)** | **Ð^{c}** |
|---|---|---|---|
| **Et-PNMVA₂₇** | 27 | 2700 | 1.23 |
| **OD-PNMVA₃₁** | 31 | 3100 | 1.21 |
| **DSPE-PNMVA₂₄** | 24 | 2400 | 1.28 |
| **DSPE-PNMVA₅₀** | 50 | 5000 | 1.27 |

| | | | |
|---|---|---|---|
| ^{a} Degree of polymerization of PNMVA calculated based on the M_{n NMR}. ^{b} Mn of PNMVA determined by ¹H NMR. determined by SEC DMF/LiBr with a PS calibration. | | | |

### 2. Preparation of lipid nanoparticles

### 2.a. By the thin film hydration method

Cationic liposomes were prepared from a mixture of 1,2-dioleoyl-3-trimethylammonium-propane (DOTAP), cholesterol and 1,2-dioleoyl-sn-glycero-3-phosphoethanolamine (DOPE) at the molar ratio 1/0.75/0.5, by the hydration of lipid film method. DOTAP and DOPE were purchased from Avanti Polar Lipids, Inc. (Alabaster, AL, USA). Cholesterol (Chol) was purchased from Sigma Aldrich (Belgium). Lipids were dissolved in chloroform or ethanol at a total concentration of 5.5 mM. Chloroform/ethanol was removed using a rotary evaporator on reduced pressure at 30 °C for 1 h. The resulting thin lipid film was hydrated with 2 mL of RNAse free water (ThermoFisher Scientific, Walthman, MA, USA), vortexed and directly extruded (Lipex^{™} Extruder, Tansferra Nanosciences Inc., Burnaby, Canada) through polycarbonate membranes (5 times on pore size of 400 nm and 10 times on pore size of 200 nm).

The cationic liposomes were then complexed to anionic siRNA by spontaneous charge interaction to form lipoplexes. Liposomes and siRNA were mixed during 30 min at a N/P ratio of 2.5, N and P corresponding respectively to the number of moles of positive (DOTAP) and negative (siRNA) charges. siRNA targeted enhance green fluorescente protein (EGFP) (siGFP), an irrelevant siRNA directed against non-human luciferase gene (siGL3) and fluorescent negative control siRNA (siGL3 Cy 5 and Cy 5.5) were provided by Eurogentec^{®} (Eurogentec SA, Liège, Belgium). The corresponding sequences are the following: siGFP: sense strand: 5'-GCAAGCUGACCCUGAAGUUC55-3', antisense strand: 5'-GAACUUCAGGGUCAGCUUGC55-3'; siGL3: sense strand: 5'-CUUACGCUGAGUACUUCGAUU55-3', antisense strand: 5'-AAUCGAAGUACUCAGCGUAAG55-3'; siGL3 Cy5 and Cy5.5 are siGL3 chemically conjugated with Cy5^{®} or Cy5.5^{®} dyes at the 5'-end of the sense strand.

To obtain the so-called "grafted lipoplexes" through a post-insertion, DSPE-PNMVA₂₄ and DSPE-PNMVA₅₀ polymers were added in different proportions to the lipoplexes obtained (5, 10, 15, 30 and 40 mol% to total lipids) under stirring in RNAse-free water (1 mM). The resulting mixture was vortexed for 15 seconds and maintained 1 h at 37°C. DSPE-PEG (1,2-distearoyl-sn-glycero-3-phosphoethanolamine-N-[methoxy(polyethylene glycol)-2000] purchased from Avanti Polar Lipids, Inc., (Alabaster, AL, USA) was used as a positive control.

### 2.b. By rapid mixing/microfluidics

A pH sensitive (SM-102) or a switchable lipid (CSL3), DSPC, Chol and DSPE-PNMVA₂₄ or DSPE-PNMVA₅₀ polymers were dissolved in ethanol (1 mM total lipid concentration) in a molar ratio of 50/10/37.5/2.5 (0.5 mL) then were mixed with a siRNA solution (1.5 mL) prepared in 25 mM of acetate buffer at pH 4 (SM-102 formulations) or prepared in PBS buffer pH 7.4 (CSL3 formulations) at an N/P ratio of 4. Mixing occurred following a rapid-mixing method which used two syringe pumps (Chemyx Fusion 200-X, KR Analytical Ltd., Sandbach, UK) connected by a T-junction (PEEK^{™} Tee, ThermoFisher Scientific [Walthman, MA, USA]) and Tube Peek (1/16", 0.010" between the pump and the T-junction, 1/16" 0.020" after the T-junction). Mixing occurred at a total flow rate of 12 mL/min with a flow rate ratio of 3:1 (aqueous to ethanol). The lipid nanoparticles were then dialyzed overnight against PBS buffer at pH 7.4, at 4 °C, and under magnetic stirring. Pur-A-LyzerTM Maxi dialysis tubes MWCO 12-14 kDa (Sigma-Aldrich, St. Louis, MO, USA) were used.

### 3. Physicochemical properties of lipoplexes

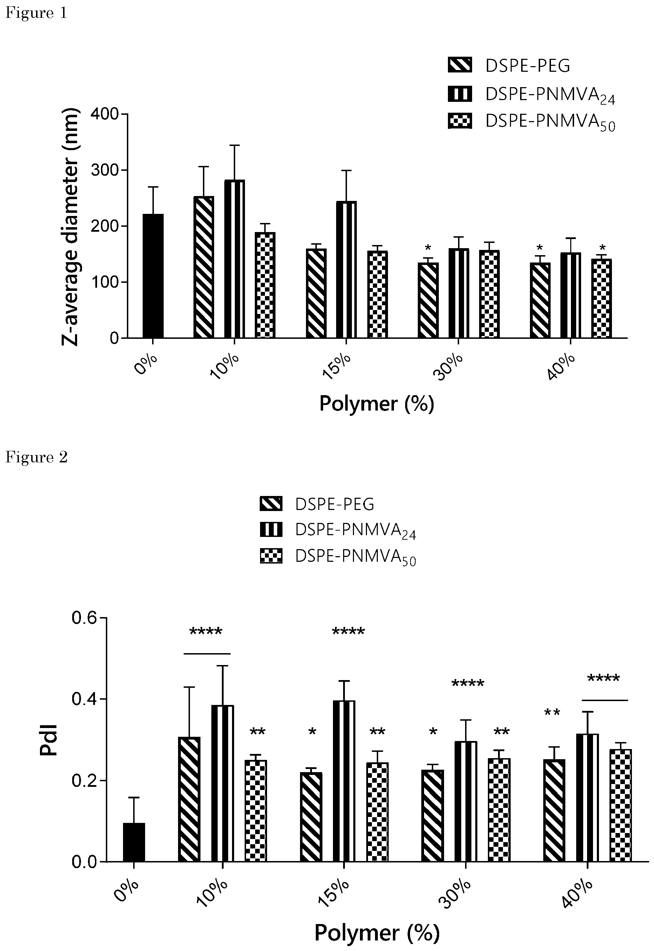

The insertion of the hydrophobic segment of the amphiphilic polymers into lipoplexes was evaluated by measuring several physicochemical properties of the grafted lipoplexes. The Z-average size (nm) and the polydispersity index (PdI) of lipoplexes were determined by Dynamic Light Scattering (DLS) using the Malvern Zetasizer^{®} (Nano ZS, Malvern Instrument, UK) in RNAse-free water with a fixed angle of 90°. The zeta potential (Zp) (mV) was determined with the same instrument. All experiments were done in triplicate (n=3) at 25°C. Each value in figures 1 to 4 represents the mean +/- standard deviation of the three independent experiments. Statistical analyses were performed by one-way ANOVA, followed by the Dunnett test. Two polymer derivatives according to the invention were tested: DSPE-PNMVA₂₄ and DSPE-PNMVA₅₀. Lipoplexes grafted by DSPE-PEG was also tested as a comparative example. Each was tested with different loading of polymer derivative/PEG versus total lipid (10, 20, 30 and 40 % in molar ratio).

The Z-average size of naked lipoplexes was around 200 nm and did not further increase significantly with increasing amounts of the different polymers (Figure 1). Although a trend of decreasing size was observed with increasing polymer concentration. The size of lipoplexes was therefore slightly affected by the addition of polymers except when large quantities of DSPE-PNMVA were added, a phenomenon also observed for DSPE-PEG.

The polydispersity index (PdI) of lipoplexes (below 0.1 for naked lipoplexes) increased significantly for DSPE-PNMVA lipoplexes as well as for PEGylated lipoplexes (PdI > 0.2 at 10, 15, 30 and 40% of DSPE-PNMVA and DSPE-PEG polymers), reflecting a higher heterogeneity of the dispersion after the insertion (here a post-insertion). We showed that the PdI of grafted lipoplexes is affected by the addition of polymers containing the hydrophobic DSPE segment (DSPE-PEG and DSPE-PNMVA) which can be due to an effective insertion within the membrane of the lipoplexes (**Figure 2**).

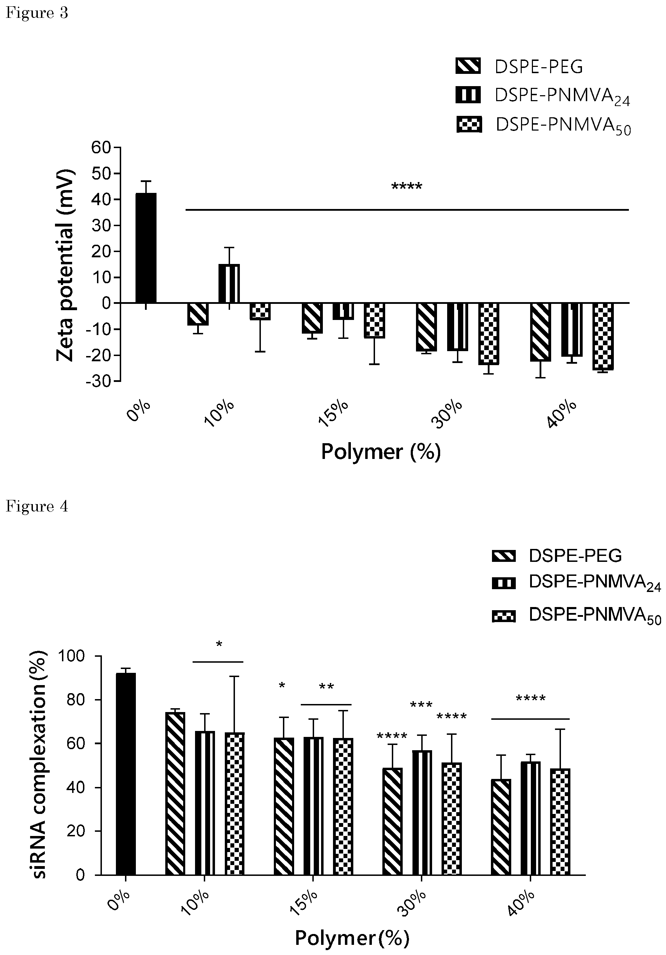

The surface charge (Zp) of all formulations was also evaluated (**Figure 3**). A significant decrease of the Zp was observed for all formulations compared to naked lipoplexes (around + 45 mV). At 40%, DSPE-PNMVA lipoplexes showed a negative Zp (around -15 mV) as observed with PEGylated lipoplexes. These results confirm the efficient insertion of the hydrophobic segment of the polymer derivatives in the lipoplexes.

The effect of polymer grafting on siRNA complexation efficiency was evaluated. The uncomplexed siRNA was quantified using a Quant-it^{™} RiboGreen^{®} RNA assay (Invitrogen^{™} (ThermoFisher Scientific, Walthman, MA, USA)) according to the manufacturer's instructions. Samples of 100 nM siRNA and siRNA calibration curves were prepared as follows. 100 µl of RiboGreen^{®} reagent was added to each well and fluorescence was measured with the FlexStation 3 Multi-Mode Microplate Reader (Molecular Devices, CA, USA). Wavelengths of excitation and emission were 485 and 530 nm, respectively. The detected fluorescence was calculated related to the concentration of free siRNA according to the blank and the calibration curves. The level of complexed siRNA was then determined.

Naked lipoplexes showed a complexation rate higher than 90% while the complexation rate observed with DSPE-PNMVA lipoplexes dropped to around 45% in the presence of 40% of these polymers, regardless of the hydrophilic chain length (**Figure 4**). Interestingly, similar results were previously observed with PEGylated lipoplexes. No significant impact of the hydrophilic chain length on size, PdI, surface charge and encapsulation efficiency was observed.

### 4. Membrane interaction

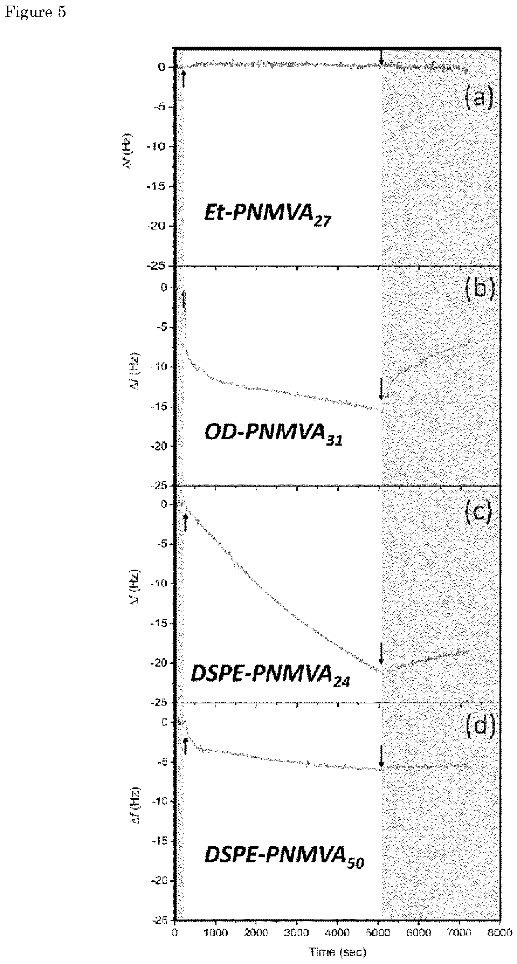

To gain insight into the interactions of the different PNMVA derivatives with lipoplexes, we monitored their binding with a model lipid bilayer via quartz crystal microbalance with dissipation analysis (QCM-D). The liposome membrane was mimicked by a supported lipid bilayer (SLB) consisting of DOTAP/Chol/DOPE deposited onto gold coated quartz crystals. QCM-D analyses of the polymer/membrane interactions were done as follows. Gold coated sensors (Q-Sense AT-cut quartz crystals, fundamental frequency of 5 MHz) were cleaned and functionalized by mercaptopropionic acid. DOTAP/Chol/DOPE (1/0.75/0.5 molar ratio) liposomes were prepared as described above except that the lipid film was rehydrated with high salt PBS buffer (20 mM PBS and 100 mM NaCl) instead of RNAse free water (final liposome concentration of 5.5 mM). Deposition of the DOTAP/Chol/DOPE membrane onto the sensor was achieved following the membrane deposition method. The sensor was exposed successively to high salt PBS buffer (20 mM PBS and 100 mM NaCl), liposome solution (0.1 mM in high salt PBS), high salt PBS buffer to remove excess of liposomes, low salt PBS buffer (20 mM PBS and 30 mM NaCl) to break the liposomes membranes by osmotic pressure, high salt PBS buffer to remove weakly bound lipids. Proper deposition of the DOTAP/Chol/DOPE membrane (44.44%/33.33%/22.22%) was confirmed by a Δ*f* of about 14 Hz. The accordingly modified sensor surface was then exposed to a continuous flow of solutions of the different PNMVA derivatives namely (a) Et-PNMVA₂₇ used a comparative example, (b) OD-PNMVA₃₁, (c) DSPE-PNMVA₂₄ and (d) DSPE-PNMVA₅₀ (30 µM in high salt PBS buffer). Sensor frequency changes due to polymer binding were converted into deposited masses according to the Sauerbrey equation (Δm = -C.Δ*f*/n) (**Figure 5**). Arrows designate the polymer solution addition (arrow up) and the final rinsing with buffer (arrow down). Grey regions correspond to buffer elution periods.

No frequency change, so no adsorption, was observed when the lipidic membrane was exposed to Et-PNMVA₂₇, which confirmed the absence of membrane interaction for PNMVA derivatives deprived of a sufficiently long aliphatic chain.

OD-PNMVA showed transient interaction with the lipidic membrane through its hydrophobic segment which is an aliphatic chain of 18 carbon atoms. DSPE showed high interaction with the lipidic membrane through its hydrophobic segment which has two aliphatic chains at its extremity. Indeed, most of DSPE-PNMVA₂₄ remained at the membrane's surface upon rinsing, corresponding to a polymer deposition of 45 ng.cm⁻². An impact of the molar mass of the PNMVA segment was observed as shorter PNMVA chains allowed better insertion of the hydrophobic segment of the polymer derivative into the lipidic membrane.

These observations corroborated the DLS, Zp and NTA measurements suggesting a significant change in the structure and charges of the lipoplexes grafted with DSPE-PNMVA.

### 5. Protein corona formation

Protein corona formation was evaluated by Nanoparticle Tracking Analysis (NTA). NTA measurements were performed using a NanoSight NS300 (Malvern Instruments Ltd., UK) equipped with a 642 nm red laser module. The protocol was inspired by Karim *et al*.⁶⁰ Lipoplexes were mixed with heat inactivated fetal bovine serum (FBS) (Gibco (Invitrogen^{™}, ThermoFisher Scientific, Walthman, MA, USA)) at 2:1 ratio (v/v) and incubated for 2 hours at 37°C under stirring process. The FBS concentration was chosen to mimic physiological conditions. The formation of the protein corona around the lipoplexes was assessed by measuring the evolution of the particles mean size from time 0 to 2 hours after the addition of FBS. Samples were measured in triplicate (n=3) at 25°C with the red laser and syringe pump speed of 40 (arbitrary unit).

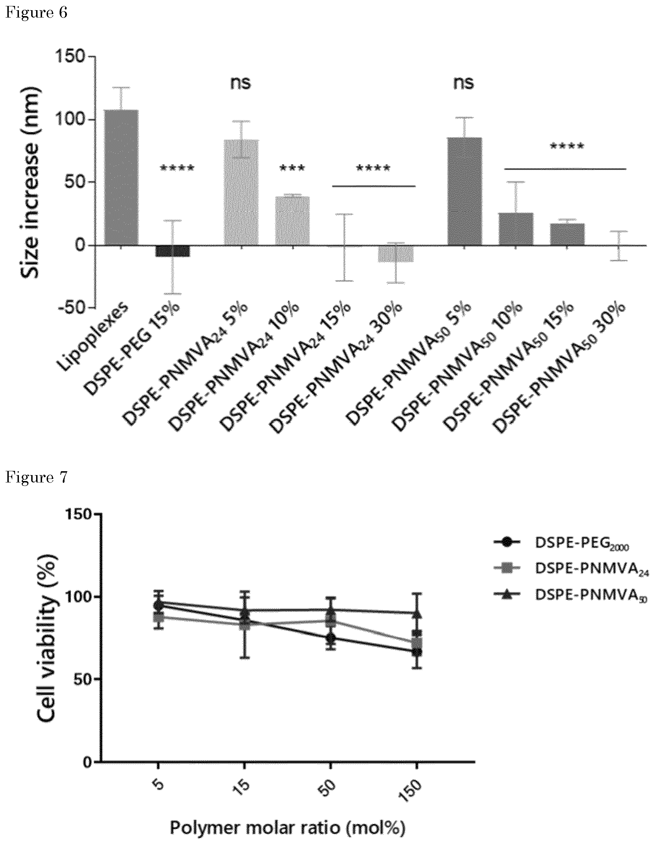

For naked lipoplexes, the formation of a protein corona resulted in changes in the distribution profile characterized by an increase in particle mean size (shift of the curve towards larger size). On the contrary, DSPE-PNMVA and DSPE-PEG lipoplexes showed less disturbed and more superimposed size distribution profiles compared to the initial profile, showing that the adsorption of FBS proteins was strongly reduced. These observations confirm that DSPE-PEG and DSPE-PNMVA were effective in preventing protein corona formation around the lipoplexes. **Figure 6** shows the size increase of naked or PNMVA derivatives-lipoplexes after 2 h in FBS Each value represents the mean +/- standard deviation (SD) of three independent experiments (n=3). For naked lipoplexes, an increase in the size was observed (about 100 nm) while for DSPE-PNMVA polymers, an amount of 15% enables to effectively avoid an increase in lipoplexes size and thus avoid the formation of the protein corona as also observed with 15% DSPE-PEG. These results confirmed the importance of the hydrophobic segment to efficiently insert into the lipoplexes and consequently protect particles from the formation of the protein corona.

### 6. Toxicity evaluation

The toxicity of polymer derivatives of the invention and of DSPE-PEG as a comparative example was evaluated in *cellulo* using MTT assay (**Figure 7**). A549 cells were treated with different amounts of polymers up to a molar ratio of 150. (p = 0.6682 for Lipoplexes; *p* < 0.001 for DSPE-PEG, *p* = 0.9979 for DSPE-PNMVA₂₄, *p* < 0.001 for DSPE-PNMVA₅₀). Each value represents the mean +/- standard deviation (SD) of three independent experiments (n=3). For MTT test, each independent experiment represents the mean of a quadruplicate. Statistical analyses were performed using a one-way ANOVA test and where each group was compared to the group untreated cells.

All DSPE-PNMVA polymers did not show cell mortality (> 80% of viable cells after 24 h of treatment) and were comparable to DSPE-PEG for all tested amounts.

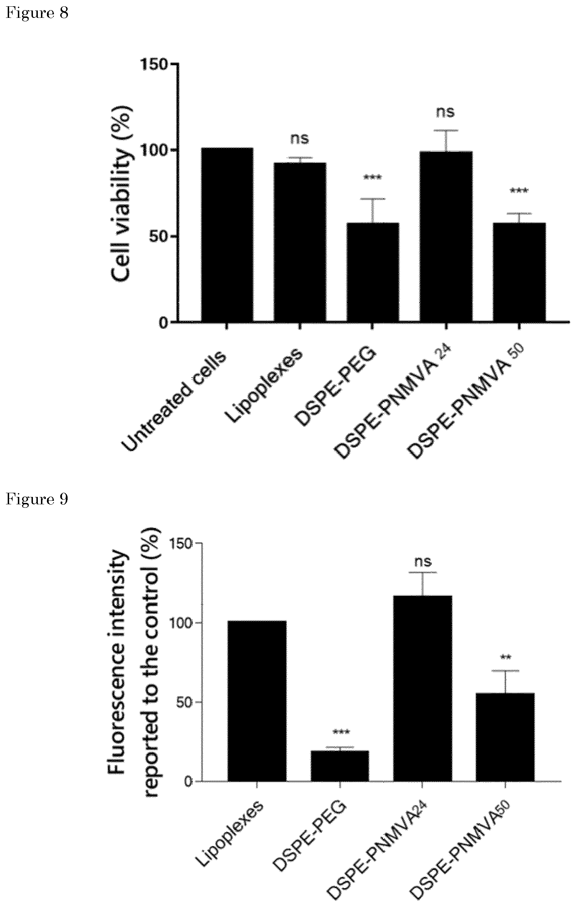

Next, cell viability was assessed with grafted lipoplexes containing irrelevant GL3 siRNA (**Figure 8**). No significant decrease in cell viability was observed for cells treated with naked lipoplexes and PNMVA₂₄ grafted lipoplexes. A significant decrease in cell viability (≈ 40%) was observed with lipoplexes grafted with DSPE-PEG and DSPE-PNMVA₅₀. However, it is important to note that the viability remained comparable between cells treated with DSPE-PEG lipoplexes and those treated with DSPE-PNMVA₅₀ lipoplexes.

Human lung adenocarcinoma cells A549 were obtained from the American Type Culture Collection (ATCC, University Blvd, Manassas, VA, USA) and A549 stably expressing Green Fluorescence Protein (GFP) (A549/GFP) were maintained in Dulbecco's modified Eagle medium (DMEM) (Biowest, VWR, Leuven, Belgium) supplemented with 10% FBS and 1% of PenStrep^{®} (Gibco (Invitrogen^{™}, ThermoFisher Scientific, Walthman, MA, USA)) at 37°C in 5% CO₂-humidified atmosphere. A549 cells were seeded in 96-well plates (5 × 10³ cells/well). After 24 h, cells were incubated for 4 h with different concentrations of polymers (different molar ratios from 5 to 150 mol% compared to the total of lipid used in the lipoplexes formulations) or lipoplexes (naked and grafted lipoplexes) in Opti-MEM^{™} (Gibco (Invitrogen^{™}, ThermoFisher Scientific, Walthman, MA, USA)) prepared at a concentration of 100 nM of GL3 siRNA. Then, the formulations were replaced by fresh culture medium containing 10% (v/v) MTT (3-(4,5-Dimethylthiazol-2-yere)-2,5-Diphenyltetrazolium Bromide) reagent (Invitrogen^{™} by ThermoFisher Scientific, Bleiswijk, Netherlands), for 3 h at 37°C. Non-treated cells were used as negative control and cells treated with hydrogen peroxide solution (H₂O₂, Sigma-Aldrich Chimie GmbH) at 20 mM were used as positive control. The absorbance was measured at 450 nm using MikroWin 2010 software (Labsis Laborsysteme GmbH, Neunkirchen-Seelscheid, Germany) on a TriStar²S LB 942 multimode reader (Berthold Technologies, Vilvoorde, Belgium). The number of viable cells is directly proportional to the absorbance value.

### In vivo Acute Toxicity in Zebrafish Model

The toxicity of the DSPE-PNMVA lipoplexes was tested on Zebrafish, a widely used model to elucidate the *in vivo* behavior (toxicity, biodistribution, pharmacokinetic, therapeutic effect) of nanoparticles.

Adult zebrafish (Danio rerio) were maintained while fulfilling the criteria of the Ethical Committee for the Use of Laboratory Animals at the University of Liège (Protocol #21-2313). Fertilized eggs were collected, washed with E3 medium, and placed in petri dishes. Fertilized eggs were incubated at 28°C and maintained on a 14 h day/10 h night period throughout the experiment. Embryos were collected at 24 hours post-fertilization (hpf), their chorions were removed and the toxicity of lipoplexes was evaluated. 10 embryos were used per condition in a 12-well plate. Embryos were treated once a day for 2 days and were observed daily for up to 72 hpf. Each well contained 1.5 ml of E3 medium containing 100 µl of naked lipoplexes or grafted lipoplexes (15% polymer, 1 and 10g siRNA GL3/kg). E3 medium stock solution is composed of 34.8 g NaCl, 1.6 g KCl, 5.8 g CaCl₂·2H₂O, 9.78 g MgCl₂·6H₂O for a final volume of 2 L of H₂O. The pH is adjusted to 7.2 with NaOH. The E3 stock solution is diluted 60x before use and 100 µL of 1% methylene blue are added. Treatment dose was replaced once daily for 2 days, and embryos were observed each day until 72 hpf.

After 2 doses, no specific toxicity was observed compared to the controlError! Reference source not found. The morphology of embryos looked normal at any concentration tested: non-curved tail or body axis deformation, no pericardial or yolk sac edema, normal embryonic development compared with the control. Based on these results, it can be concluded that the DSPE-PNMVA lipoplexes are not toxic in these live organisms.

To plan parenteral administration, the blood biocompatibility of lipoplexes was evaluated. Hemolysis was tested based on Drabkin method. Washed red blood cells from 3 healthy donors were incubated with lipoplexes for 1 h at 37°C or room temperature (RT) in presence of 5% CO₂. After centrifugation, the supernatant is collected, and hemoglobin concentration is determined by spectrometric detection of cyanmethemoglobin at 540 nm and expressed in percentage compared to Triton X-100 (inducing 100% of hemolysis). Platelet aggregation was tested using a protocol previously described and using a final concentration of lipoplexes of 200 nM siRNA. Polymer derivatives were tested at 15% molar ratio of total lipids. The study protocol was in accordance with the Declaration of Helsinki and was approved by the Medical Ethical Committee of the Centre Hospitalier Universitaire (CHU), UCL Namur (Yvoir, Belgium).

Compared with the control (Triton X-100), DSPE-PNMVA (15%) lipoplexes did not show a significant hemolytic or platelet aggregation effect. All these results suggest the safe use of DSPE-PNMVA polymers as a PEG alternative. DSPE-PNMVA was found hemocompatible.

### 7. Cell uptake and siRNA efficiency

One critical step for an efficient gene silencing is the siRNA internalization and release. This step is considered as one of the most important limitations of PEGylated lipoplexes as polymer grafting is known to reduce interactions with cell membranes and subsequently decrease cellular uptake and endosomal escape preventing siRNA delivery and gene silencing.

To evaluate the capacity of grafted lipoplexes to cross the cell membrane and gain access to cytoplasm (cellular internalization), the intracellular fluorescence intensity of labeled-Cy5 GL3 siRNA-lipoplexes was measured. For this, A549 cells were seeded in 24-well plates (1 × 10⁵ cells/well). After 24 h, lipoplexes prepared with 100 nM of siRNA conjugated to Cy5 were added to cells for 4 h. Non-treated cells were used as negative control. After treatment, cells were washed with PBS, harvested with Trypsin-EDTA^{®} and re-suspended in PBS. 1×10⁴ cells were analyzed by BD FACS Canto^{™} II flow cytometer (BD Biosciences, Franklin Lakes, USA) to measure the intracellular fluorescence of Cy5.

As shown in **Figure 9****,** compared to naked lipoplexes, the cell internalization of lipoplexes was reduced by about 80% after the grafting of DSPE-PEG on their surface. This observation is consistent with the well-known PEG dilemma. Compared with naked lipoplexes, the presence of DSPE-PNMVA₂₄ polymer did not reduce the amount of internalized fluorescent siRNA. With DSPE-PNMVA₅₀ polymer, cellular penetration of lipoplexes was reduced by about 50%. Therefore, for DSPE-PNMVA polymer, cell uptake seems to be dependent on the length of the hydrophilic chain. Indeed, the intracellular fluorescence intensity decreased with the long hydrophilic chain. A lower uptake was observed when cells were treated with lipoplexes grafted with DSPE-PNMVA₅₀ polymer compared to DSPE-PNMVA₂₄. In all cases, the internalization of DSPE-PNMVA grafted lipoplexes was higher than that of DSPE-PEG grafted lipoplexes. DSPE-PNMVA₂₄ and DSPE-PNMVA₅₀ lipoplexes have about 6- and 3-fold higher cellular uptake than DSPE-PEG lipoplexes, and thus appear to be less prone to the dilemma effect than PEG.

The impact of the polymer on lipoplexes gene silencing efficiency was evaluated. To address this question, A549 cells stably expressing GFP were used and the ability of naked lipoplexes and grafted lipoplexes to transfect a siRNA against GFP was determined. A549/GFP cells were seeded in 6-well plates (1.8 × 10⁵ cells/well). After 24 h, cell culture medium was replaced by 1 mL of Opti-MEM^{™} containing lipoplexes and grafted lipoplexes prepared with 100 nM of siRNA EGFP (siGFP). Lipofectamine^{®} RNAiMAX (ThermoFisher Scientific, Walthman, MA, USA) associated with EGFP siRNA was used as positive control. Cells were incubated for 4 h at 37°C and then, the formulations were replaced by fresh culture medium. After 72 h, cells were washed with PBS pH 7.4, harvested with Trypsin-EDTA^{®} and re-suspended in 300 µL of PBS. 1 × 10⁴ cells were analyzed by CytoFLEX flow cytometer to measure the GFP fluorescence intensity of A549 cells. Intracellular fluorescence intensity of siRNA GL3 Cy5 was detected by flow cytometry after 4 h of incubation.

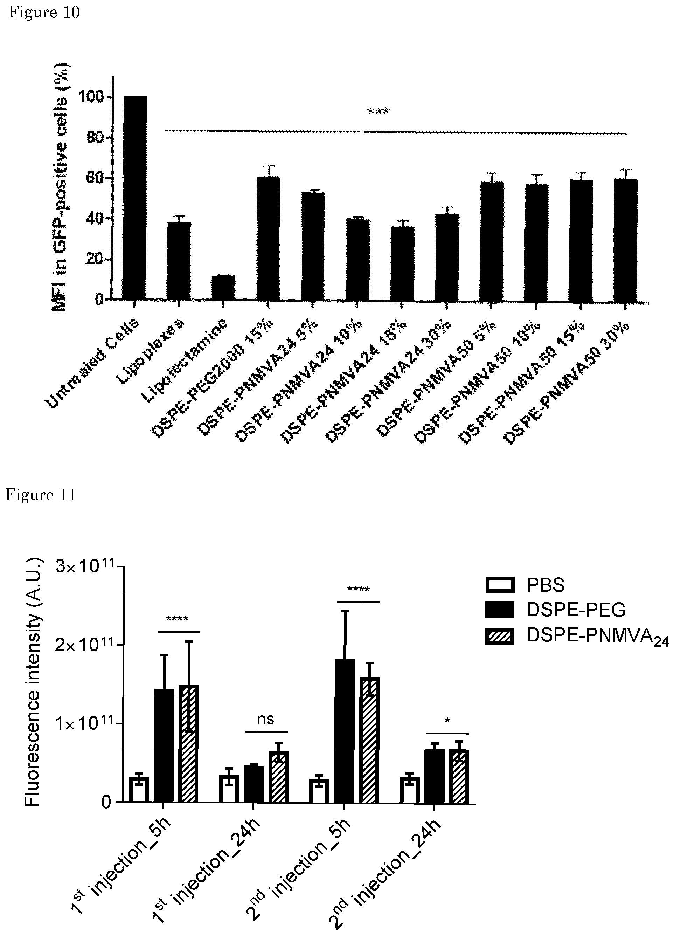

The inhibition of the GFP fluorescence intensity is an indicator of siRNA transfection efficiency, which combines cellular entrance, delivery/release, and degradation of target mRNA. As shown in **Figure 10**, naked lipoplexes decreased the fluorescence by about 60% after 72 h. Due to the PEG dilemma, PEGylated lipoplexes decreased the fluorescence less than naked lipoplexes (about 40%). Interestingly, DSPE-PNMVA lipoplexes efficiently decreased the fluorescence (fluorescence decrease comprised between 40% and 65%). This transfection efficiency seems to be correlated to the cell uptake too. Even if the difference is not statistically significant, an increase in the hydrophilic chain length tends to decrease the efficiency as DSPE-MNMVA₂₄ lipoplexes (15%) decreased the fluorescence for about 60% while DSPE-PNMVA₅₀ lipoplexes (15%) decreased the fluorescence for about 40%. Furthermore, with respect to the impact of the polymer percentage, the best polymer concentration seems to be 15% as higher percentage seems to have a negative effect. Lipoplexes grafted with 15% DSPE-PNMVA₅₀ or DSPE-PEG give similar gene delivery efficiency while DSPE-PNMVA₂₄ lipoplexes (15%) were able to decrease the fluorescence for about 60%. Compared to DSPE-PEG, DSPE-PNMVA₂₄ 15% avoids the formation of a protein corona around lipoplexes while ensuring more efficient siRNA delivery.

### 8. In vivo evaluation in mice

Considering the physicochemical results, the low toxicity as well as the good internalization and efficiency to silence target mRNA, lipoplexes grafted with 15% DSPE-PNMVA₂₄ were selected and compared with DSPE-PEG in immunocompetent BALB/c mice.

Female BALB/c mice aged 8 weeks (20-25 g) were purchased from Janvier Labs (Saint-Berthevin, France). All animal experiments were evaluated and approved by the Animal and Ethics Review Committee of the University of Liege (Protocol #21-2397). During the study, animals were housed in a controlled climate and photoperiod (12 h light-dark cycles) and had free access to water and food. 3 groups with 8 mice/group were divided as following: (i) a control group injected with PBS, (ii) a group injected with DSPE-PEG lipoplexes, and (iii) a group injected with DSPE-PNMVA₂₄lipoplexes prepared at a concentration of 1 mg/kg of siGL3 Cy5.5. The *in vivo* biodistribution and ABC phenomenon in mice were investigated after intravenous injection of 100 µl of grafted lipoplexes (15% DSPE-PEG and DSPE-PNMVA₂₄ polymer) containing 1 mg/kg of control siGL3 Cy5.5 at N/P 2.5 and isotonized with mannitol, once a week for two weeks. *In vivo* fluorescence imaging was performed on live animals 5 h and 24 h after each injection using Living Image^{®} software in IVIS Spectrum *In Vivo* Imaging System (PerkinElmer). One week after the second injection, mice were sacrificed, and the main organs (liver, kidney, heart, spleen, and lung) were removed to perform *ex-vivo* fluorescence imaging.

**Figure 11** shows such biodistribution with *p* < 0.0001 for DSPE-PEG and DSPE-PNMVA₂₄ after 5 h of the 1^{st} and the 2^{nd} injection, *p* = 0.6929 for DSPE-PEG and *p* = 0.0852 for DSPE-PNMVA₂₄ after 24 h of the 1^{st} injection, *p* = 0.0429 for DSPE-PEG and *p* = 0.0435 for DSPE-PNMVA₂₄ after 24 h of the 2^{nd} injection. A significant fluorescence intensity was detected in mice injected with both formulations 5 h after the first and the second injection of DSPE-PEG or DSPE-PNMVA₂₄ lipoplexes. 24 h after each injection, the fluorescence decreased, but for the 24 h after the second injection, mice injected with DSPE-PEG and DSPE-PNMVA₂₄ lipoplexes displayed a weak but significant fluorescent signal. This result shows that lipoplexes would circulate a bit longer after the second injection which tends to show that there is less ABC phenomenon for these formulations.

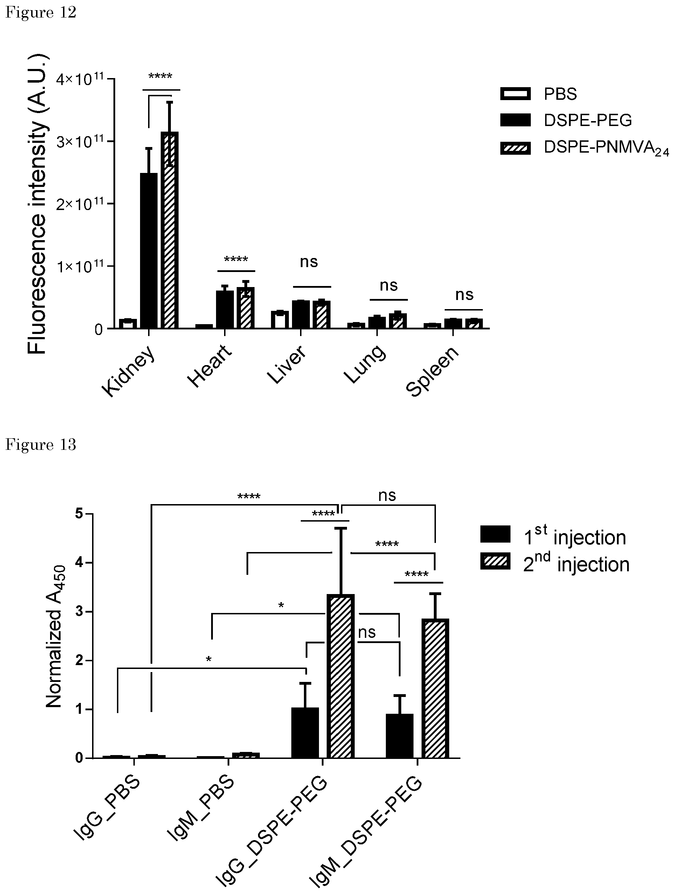

**Figure 12** shows a post-mortem investigation of the biodistribution of DSPE-PEG and DSPE-PNMVA₂₄ lipoplexes on mice's organs including liver, spleen, heart, lung, and kidney, with *p* < 0.0001 for DSPE-PEG and DSPE-PNMVA₂₄ in kidney and heart, *p* = 0.1668 for DSPE-PEG in liver, *p* = 0.1623 for DSPE-PNMVA₂₄ in liver, *p* = 0.5622 for DSPE-PEG in lung, *p* = 0.2249 for DSPE-PNMVA₂₄ in lung, *p* = 0.7348 for DSPE-PEG in spleen and *p* = 0.7319 for DSPE-PNMVA₂₄ in spleen.

The quantification of the fluorescence intensity showed a high signal of Cy5.5 of DSPE-PEG and DSPE-PNMVA₂₄ lipoplexes in kidney. A weak fluorescence was also detected in the heart whereas the other organs (liver, lung, and spleen) were completely negative. The absence of hepatic accumulation for both formulations suggests good stealth properties of both DSPE-PEG and DSPE-PNMVA₂₄ lipoplexes formulations, which do not seem to be cleared by the MPS (mononuclear phagocyte system). However, both lipoplexes formulations were found to be mainly trapped in the kidney suggesting nanoparticles-kidney interactions and renal clearance. The abilities of DSPE-PEG and DSPE-PNMVA₂₄ lipoplexes to either fully experience renal clearance or accumulate in certain parts of the kidney probably relies on a combination of various physicochemical characteristics such as their diameters, surface charge, structural properties, morphology.

### Detection of IgG and IgM anti-PEG or anti-PNMVA Antibodies and Pro-Inflammatory Cytokines

Blood was collected from mice tails on day 7 after the 1^{st} injection and by cardiac puncture on day 14. To obtain serum, the blood was placed at room temperature for 30 min and then centrifuged at 1000 g at 4°C for 15 min. The serum collected from mice injected with PBS was used as a negative control. A direct ELISA procedure was employed to detect PEG- or PNMVA-specific IgM and IgG antibodies in the serum. 2 µg of DSPE-PEG or DSPE-PNMVA₂₄ in ethanol was added to 96-well plates. Coated plates were allowed to completely air dry. The plates were then washed with PBS and then blocked for 1 h with PBS containing 2% BSA. Diluted serum samples (1: 1000) were then added in wells, incubated for 1 h and washed five times with PBS. Horseradish peroxidase (HRP)-conjugated antibody diluted 1/1000 in PBS (Goat anti-mouse IgM-HRP (Invitrogen^{™}, Camarillo, USA) or Goat anti-mouse IgG-HRP (Cell Signaling, Leiden, The Netherlands)) were added in wells. After 45 min, wells were washed five times with PBS. The coloration was initiated by adding TMB (Invitrogen^{™}). After 30 min, the reaction was stopped by adding sulfuric acid 2 M. The absorbance was measured at 450 nm using MikroWin 2010 software (Labsis Laborsysteme GmbH, Neunkirchen-Seelscheid, Germany) on a TriStar²S LB 942 multimode reader (Berthold Technologies, Bad Wildbad, Germany). All incubations were performed at room temperature using a plate shaker (300 rpm).

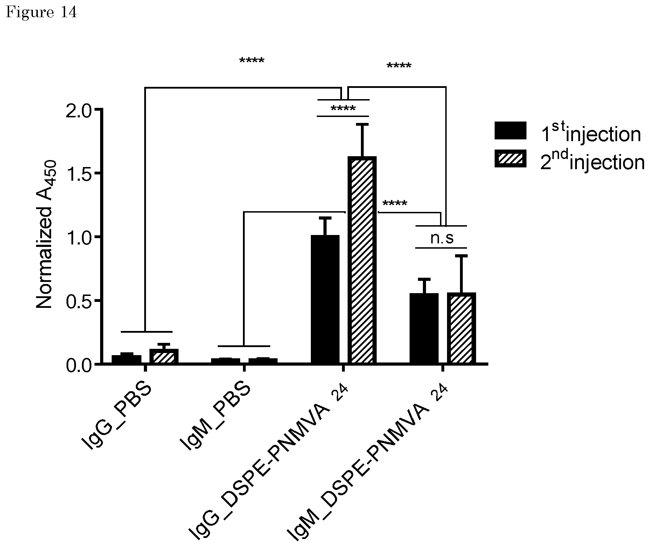

The IgG level, which relies on IgM stimulation is an important index of immune response when foreign materials are injected into the body. The level of IgM and IgG against DSPE-PEG or DSPE-PNMVA₂₄ were evaluated in mice's serum one week after each injection using ELISA assay and is shown in **Figure 13** and **Figure 14** respectively. Results were compared to a negative control group injected with PBS. For ELISA assay, the result of each mouse represents the mean of a duplicate. Statistical analyses were performed by two-way ANOVA test.The production of IgG and IgM directed against DSPE-PEG or DSPE-PNMVA₂₄ was observed after the first injection. After the second injection, the level of anti-DSPE-PEG IgM and IgG further increased, with around 3-fold induction compared to the first injection. This higher presence of detectable anti-PEG IgG after the second injection implies that immunological memory likely exists. Such rapid elevation of anti-PEG antibodies levels upon repeated injections of liposomes could pose a real challenge for individuals by inducing severe adverse reactions. After the second injection with DSPE-PNMVA₂₄ lipoplexes, a slight significant increase (around 1.5-fold) was observed for anti-DSPE-PNMVA₂₄ IgG, but no increase of anti-DSPE-PNMVA₂₄ IgM was detected, compared to the first injection. This result shows that DSPE-PNMVA₂₄ lipoplexes are less immunogenic than DSPE-PEG lipoplexes.

Aiming at studying functions related to inflammation, we finally investigated the effect of DSPE-PEG or DSPE-PNMVA₂₄ on the systemic production of two pro-inflammatory cytokines (TNFα, IL-1beta) following the second intravenous injection using commercial ELISA kits (Mouse IL-1 beta ELISA kit and Mouse TNF-alpha ELISA kit; Invitrogen^{™}, Vienna, Austria). The dosage was performed according to manufacturer's procedures. Levels of IL-1β and TNF-α were measured *in vivo.*

No increase in the level of IL-1β and TNF-α was detected in the serum of mice injected with DSPE-PEG lipoplexes or DSPE-PNMVA₂₄ lipoplexes compared to the PBS control group (p = 0.555 for DSPE-PEG and p= 0.3847 for DSPE-PNMVA₂₄) and TNF-α (p = 0.5865 for DSPE-PEG and *p* = 0.9226 for DSPE-PNMVA₂₄), each value representing the mean +/- standard deviation (SD) of at least 7 mice (n = 7 or 8). This result indicates that the intravenous administration of these grafted lipoplexes did not induce inflammation-related responses *in vivo.* In accordance with the lack of toxicity in *vitro,* on human blood and in in *vivo* zebrafish model, these results confirmed the safety of DSPE-PNMVA₂₄ lipoplexes for *in vivo* applications.

## Claims

1. A polymer derivative comprising a hydrophilic segment obtained from the polymerisation of N-methyl-N-vinylacetamide, coupled to at least one hydrophobic segment, said hydrophobic segment comprising at least one aliphatic chain having 8 to 28 carbon atoms.

2. The polymer derivative of claim 1 wherein the at least one hydrophobic segment is selected from the group of hydrophobic segments comprising a single aliphatic chain having 8 to 28 carbon atoms, comprising two aliphatic chains having 8 to 28 carbon atoms, comprising one or two fatty acids bearing an aliphatic chain having 8 to 28 carbon atoms, comprising a diglyceride bearing two aliphatic chains having 8 to 28 carbon atoms, comprising a ceramide bearing aliphatic chains having 8 to 28 carbon atoms or comprising a phospholipid bearing aliphatic chains having 8 to 28 carbon atoms.

3. The polymer derivative of claim 1 or 2 wherein the at least one aliphatic chain is a saturated aliphatic chain.

4. The polymer derivative of any of previous claims wherein the at least one hydrophobic segment is a phosphatidylethanolamine bearing two aliphatic chains having 8 to 28 carbon atoms, preferably 12 to 24 carbon atoms, more preferably 14 to 20 carbon atoms, most preferably 16 to 18 carbon atoms.

5. The polymer derivative of any of previous claims wherein the at least one hydrophobic segment is 1,2-distearoyl-sn-glycero-3-phosphorethanolamine.

6. The polymer derivative of any of previous claims wherein the hydrophilic segment has a molecular weight of between 1 000 and 10 000 g/mol, preferably between 1500 and 6000 g/mol, even more preferably between 2000 and 4000 g/mol.

7. A method for preparation of the polymer derivative according to any of previous claims comprising the steps of:
(i) coupling a hydrophobic segment comprising at least one aliphatic chain of 8 to 28 carbon atoms to a chain controlling agent;
(ii) polymerizing N-methyl-N-vinylacetamide in the presence of said coupled chain controlling agent and optionally of an initiator of radical polymerization;
or
(i) polymerizing N-methyl-N-vinylacetamide in the presence of a chain controlling agent functionalized by a reactive group and optionally of an initiator of radical polymerization;
(ii) coupling the obtained polymer to a hydrophobic segment comprising at least one aliphatic chain of 8 to 28 carbon atoms by reaction with said reactive group.

8. The polymer derivative of any of previous claims 1-6 for use in a lipid nanoparticle.

9. A lipid nanoparticle comprising the polymer derivative of any of previous claims 1-6 in a molar percentage of from 1 to 50 % to the total of lipid, preferably from 1 to 30% to the total of lipid.

10. The lipid nanoparticle of claim 9 having a size of less than 500 nanometers, preferably less than 200 nanometers.

11. The lipid nanoparticle of claims 9 to 10 further comprising an active agent.

12. The lipid nanoparticle of claims 9 to 11 comprising a nucleic acid.

13. The lipid nanoparticle of claims 9 to 12 for use in a method of medical treatment or of prevention of a disease.

14. The lipid nanoparticle of claim 12 for use in nucleic acid delivery.

15. A pharmaceutical composition comprising the lipid nanoparticle of claims 9 to 12 and one or more pharmaceutically acceptable carrier, said pharmaceutical composition preferably selected from a vaccine formulation.
